# EUROPEAN PATENT APPLICATION

(11) **EP 4 197 560 A1**
(43) Date of publication of application: **21.06.2023**
(21) Application number: 21856277.5
(22) Date of filing: 13.08.2021
(51) Int. Cl.: A61K 47/68, A61K 38/17, A61K 38/26, A61K 38/22, A61K 47/60, A61K 47/54, A61K 9/00, A61P 1/16

(54) **PHARMACEUTICAL COMPOSITION COMPRISING A TRIPLE-ACTIVATOR PERSISTENT CONJUGATE AS ACTIVE INGREDIENT**

(30) Priority: 14.08.2020 KR 20200102604
(71) Applicant: Hanmi Pharm. Co., Ltd., Hwaseong-si, Gyeonggi-do 18536 (KR)
(72) Inventor: BAEK, Seungjae, Seoul 05545 (KR); CHOI, Jaeduk, Seoul 05545 (KR); SHIN, Wonjung, Seoul 05545 (KR); KIM, Jung Kuk, Hwaseong-si, Gyeonggi-do 18469 (KR)
(74) Representative: Mewburn Ellis LLP
(86) International application number: PCT/KR2021/010780
(87) International publication number: WO 2022/035271

(57) **Abstract**

Provided are a pharmaceutical composition including a long-acting conjugate of a triple agonist as an active ingredient and a method of treating obesity and/or a non-alcoholic fatty liver disease using the same. The pharmaceutical composition including the long-acting conjugate of the triple agonist of the present invention may be stably applied to treatment of obesity and/or a non-alcoholic fatty liver disease without side effects according to therapeutic effects on obesity and/or the non-alcoholic fatty liver disease.

## Description

### [Technical Field]

The present invention relates to a pharmaceutical composition including a long-acting conjugate of a triple glucagon/GLP-1/CIP receptor agonist as an active ingredient and a method of treating obesity and/or a non-alcoholic fatty liver disease using the same.

### [Background Art]

Obesity, caused by energy imbalance when an excess energy intake over energy expenditure continues for a long time, is a metabolic disease that affects the whole body. As a representative metabolic disease, obesity itself is a disease, but it is known to increase the risk of developing various other diseases (e.g., diabetes, hyperlipidemia, hypertension, and fatty liver).

Non-alcoholic steatohepatitis disease (NAFLD) is a type of disease exhibiting similar histological findings to those of alcoholic hepatitis, although the disease is not related to alcohol intake and includes non-alcoholic fatty liver (NAFL), non-alcoholic steatohepatitis (NASH), liver fibrosis, and hepatocellular carcinomas. With the increase in the number of patients with obesity and diabetes, non-alcoholic fatty liver disease tends to increase, and the annual incidence rate is about 16% in Korea. Various factors such as insulin resistance, obesity, lipotoxicity, and inflammatory response have been known as causes of non-alcoholic fatty liver disease. Among these, insulin resistance is the biggest cause.

In order to prevent and/or treat such non-alcoholic fatty liver diseases, intensive efforts have been made to reduce insulin resistance. For example, clinical trials for thiazolidinediones (TZD) and metformin, which are insulin sensitizers, are still being actively conducted (Hepatology (2003) 38:1008-17, J Clin Invest. (2001) 108:1167-74), and clinical trials to apply a GLP-1 receptor agonist such as Victoza, Byetta, or Ozempic to non-alcoholic fatty liver diseases have been extensively conducted. However, there is still a need to develop drugs capable of treating obesity and non-alcoholic fatty liver diseases while increasing patient convenience without side effects.

Triple agonists having activities to all of glucagon-like peptide-1 (GLP-1), glucose-dependent insulinotropic polypeptide (GIP), and glucagon receptors have been reported as drugs capable of treating metabolic syndromes including obesity with increased patient convenience and without side effects by increasing half-life thereof (WO 2017116204 A and WO 2017116205 A).

However, although the effect of a drug is confirmed based on *in vitro* experiments, there is a need to determine an administration route and dosage which are safe and effective for treatment in the case of administering the drug to a subject (in particular, humans) via clinical trials. Due to these properties, it is actually difficult to develop a drug that is suitable for commercialization via actual clinical trials.

Dose translation from animal to human studies is regarded as one of the difficulties and barriers in new drug development since it is very complicated and difficult (Reagan-Shaw S et al., Dose translation from animal to human studies revisited. Fed Am Soc Exp Biol J 2008). That is, optimal dosing intervals and doses may be determined based only on clinical trial designs and results thereof in consideration of pharmacological activities, side effects, and tolerated doses (doses for safe administration) in the human body.

For example, a patent (Patent No. 78931) related to sildenafil, the pharmaceutical substance of Viagra^{®} for treatment of erectile dysfunction, discloses doses and dosing intervals as "a dose of a compound administered to humans (average adult, 70 kg) for oral administration is generally in the range of 4 mg to 800 mg". However, sildenafil in a dose lower than 25 mg was not approved in the United States due to lack of efficacy, and sildenafil in daily doses of 25 mg, 50 mg, and 100 mg have been approved and entered the market. Among these, sildenafil in a dose of 25 mg was poor in demand due to insufficient efficacy, and sildenafil has only entered the market in Korea in doses of 50 mg and 100 mg. As can be seen from the above example, "the daily dose of 4 mg to 800 mg" suggested in the patent related to a pharmaceutical compound is a very broad range in which a difference between the minimum and maximum values is above 200-fold. That is, although the patent related to sildenafil discloses that the effects of the minimum dose of 4 mg and the maximum dose of 800 mg have no difference and both can be administered to the human body, pharmacological effects and safety thereof are not guaranteed in the above-described range. In fact, based on the results confirmed by clinical trials, efficacy was insufficient from the dose of 4 mg to 25 mg failing to treat the condition of erectile dysfunction, and side effects such as blushing and abnormal eyesight were observed in doses of over 100 mg, and thus safety cannot be guaranteed in doses over 100 mg, not to mention the maximum dose of 800 mg.

In addition, a dosing interval is determined by considering a long-lasting property of a drug and is generally estimated using a half-life. That is, when an effective amount of a drug lasts for 6 hours, it is effective to administer the drug four times a day. In general, as a dosage of a drug increases, efficacy thereof increases as well. However, side effects also increase, and thus it is not preferable to increase the dosage indefinitely. In addition, when a dosage reaches a certain level, efficacy reaches a maximum level and cannot increase any more (sigmoidal curve) in most cases, but the increasing efficacy may also decrease (biphasic effect). Therefore, it is difficult to determine the dosing interval and dose because these factors should be considered.

In this point of view, even in the case where efficacy of a drug is known in the art, a method of improving therapeutic effects of the drug, inhibiting side effects, and improving dosing convenience by using an administration method and a dose different from those known in the art may also be regarded as being as valuable as a method of developing novel pharmaceutical substances or medical uses thereof. Any method of improving dosing convenience may be of great value for patients as long as the same therapeutic effects are obtained while decreasing a dose or extending a dosing interval. It is not easy for patients to consistently take a drug every day at a particular time of day, and there are many cases in which treatment is discontinued due to difficulty of taking a drug, or in which therapeutic effects on a disease are low due to difficulty of compliance with a prescribed dosage.

Therefore, even when a triple agonist available as a medicine and having a broad effective dosage range without causing serious side effects is confirmed, there is a need to identify clinically effective administration methods and doses of the triple agonist to apply the triple agonist to actual medical treatment.

### [Disclosure]

### [Technical Problem]

The development of the clinically effective usage and dosage of the triple agonist is required.

### [Technical Solution]

An object of the present invention is to provide a composition including as an active ingredient a long-acting conjugate of a triple agonist having activities to all of a glucagon receptor, a glucagon-like peptide-1 (GLP-1) receptor, and a glucose-dependent insulinotropic polypeptide (GIP) receptor, the composition being a pharmaceutical composition for preventing or treating obesity and/or a non-alcoholic fatty liver disease, wherein the long-acting conjugate of the triple agonist is parenterally administered to a patient with obesity and/or a non-alcoholic fatty liver disease once a week at a dose of 0.5 mg to 8 mg.

### [Advantageous Effects]

The composition including the long-acting conjugate of the triple agonist of the present invention may be stably applied to treatment of obesity and/or a non-alcoholic fatty liver disease without side effects in accordance with therapeutic effects thereof on obesity and/or the non-alcoholic fatty liver disease.

### [Brief Description of Drawings]

FIG. 1 shows body weights of mice on Day 28 after a high-fat diet obesity animal model (mouse) is administered with long-acting conjugates of SEQ ID NOS: 42, 43, and 50 once every 2 days for 28 days, the body weights measured every 2 days (p<0.05, **p<0.01, ***p<0.001, vs. vehicle by one-way ANOVA).
FIG. 2 shows weights of mesenteric fat of mice on Day 28 after a high-fat diet obesity animal model (mouse) is administered with long-acting conjugates of SEQ ID NOS: 42, 43, and 50 once every 2 days for 28 days, the weights of mesenteric fat measured every 2 days (p<0.05, **p<0.01, ***p<0.001, vs. vehicle by one-way ANOVA).
FIG. 3 shows information on groups of obese patients administered with the long-acting conjugate of SEQ ID NO: 42.
FIG. 4 shows blood concentrations of the long-acting conjugate of SEQ ID NO: 42 in patients having obesity and administered with the long-acting conjugate of SEQ ID NO: 42.
FIG. 5 shows Cmax (ng/mL), Tₘₐₓ (hr), T_{1/2} (hr), AUC_{0-inf} (ng/mL.h), Dose-normalized Cₘₐₓ (ng/mL/mg), and Dose-normalized AUCinf (ng/mL.h/mg) confirmed in patients having obesity and administered with the long-acting conjugate of SEQ ID NO: 42.
FIG. 6 shows treatment emergent adverse events (TEAEs) for one month after administration of the long-acting conjugate of SEQ ID NO: 42.
FIG. 7 shows heart rate (HR), systolic blood pressure (SPB), diastolic blood pressure (DBP), and rate pressure product (RPP) measured for 4 days after the long-acting conjugate of SEQ ID NO: 42 is administered.
FIGS. 8 to 10 show immunogenicity (anti-drug antibodies (ADAbs); neutralizing antibodies (nAbs); and anti-polyethylene glycol antibodies (anti-PEG)) measured after the long-acting conjugate of SEQ ID NO: 42 is administered.
FIG. 11 shows information on groups of patients with a non-alcoholic fatty liver disease administered with the long-acting conjugate of SEQ ID NO: 42.
FIG. 12 shows blood concentrations of the long-acting conjugate of SEQ ID NO: 42 in patients having a non-alcoholic fatty liver disease and administered with the long-acting conjugate of SEQ ID NO: 42.
FIG. 13 shows Cₘₐₓ (ng/mL), Tₘₐₓ (hr), T_{1/2} (hr), and AUC₀₋₁₆₈ (ng/mL h) confirmed in patients having a non-alcoholic fatty liver disease and administered with the long-acting conjugate of SEQ ID NO: 42. W1 means Week 1 and W12 means Week 12.
FIG. 14 shows treatment emergent adverse events (TEAEs) for one month after administration of the long-acting conjugate of SEQ ID NO: 42.
FIG. 15 shows images obtained by MRI indicating reduction in visceral fat mass in the liver in patients with a non-alcoholic fatty liver disease administered with the long-acting conjugate of SEQ ID NO: 42.
FIG. 16 shows reduction in liver fat content in patients with a non-alcoholic fatty liver disease administered with the long-acting conjugate of SEQ ID NO: 42.
FIG. 17 shows reduction in liver fat content in patients with a non-alcoholic fatty liver disease administered with the long-acting conjugate of SEQ ID NO: 42.

### [Best Mode]

An aspect of the present invention provides a composition including as an active ingredient a long-acting conjugate of a triple agonist having activities to all of a glucagon receptor, a glucagon-like peptide-1 (GLP-1) receptor, and a glucose-dependent insulinotropic polypeptide (GIP) receptor.

In a specific embodiment, the composition is a pharmaceutical composition for preventing or treating obesity and/or a non-alcoholic fatty liver disease, wherein the long-acting conjugate of the triple agonist is parenterally administered to a patient with obesity and/or the non-alcoholic fatty liver disease once a week at a dose of 0.5 mg to 8 mg.

In another specific embodiment, the long-acting conjugate of the triple agonist is parenterally administered to a patient with obesity and/or a non-alcoholic fatty liver disease once a week at a dose of 2 mg to 6 mg.

In the pharmaceutical composition according to any one of the specific embodiments, the parenteral administration may be subcutaneous administration.

In the pharmaceutical composition according to any one of the specific embodiments, the patient with obesity may have a body mass index (BMI) of 23 kg/m² or more.

In the pharmaceutical composition according to any one of the specific embodiments, the patient with a non-alcoholic fatty liver disease may have a liver fat content of 8% or more measured by magnetic resonance imaging-derived proton density fat fraction (MRI-PDFF).

In the pharmaceutical composition according to any one of the specific embodiments, the conjugate is represented by Formula 1 below:

[Formula 1] X-L-F

wherein
X is a peptide including one of the amino acid sequences of SEQ ID NOS: 1 to 102;
L is a linker including an ethylene glycol repeating unit;
F is an immunoglobulin Fc region; and
- is a covalent bond between X and L and between L and F.

In the pharmaceutical composition according to any one of the specific embodiments, an individual administered with the pharmaceutical composition exhibits at least one of properties (a) to (f) below:
(a) weight loss;
(b) blood pressure decrease;
(c) visceral fat mass reduction in the liver;
(d) NAS decrease;
(e) reduction in ballooning degeneration of hepatocytes or the number of lobular inflammation; and
(f) fibrosis score decrease.

In the pharmaceutical composition according to any one of the specific embodiments, the pharmaceutical composition may be administered to an arm, thigh, or abdomen.

In the pharmaceutical composition according to any one of the specific embodiments, the F may be an IgG Fc region.

In the pharmaceutical composition according to any one of the specific embodiments, the long-acting conjugate may have a structure in which the Fc region is in a dimer form formed of two polypeptide chains and the peptide X is linked to only one of the two polypeptide chains of the Fc dimer in the long-acting conjugate.

In the pharmaceutical composition according to any one of the specific embodiments, the polypeptide chain of the Fc dimer may include an amino acid sequence of SEQ ID NO: 123.

In the pharmaceutical composition according to any one of the specific embodiments, a ring may be formed between the 16^{th} and 20^{th} amino acids from the N-terminus of X.

In the pharmaceutical composition according to any one of the specific embodiments, the X may include an amino acid sequence selected from the group consisting of SEQ ID NOS: 21, 22, 27, 30 to 32, 34, 36, 37, 42, 43, 50 to 56, 58, 64 to 80, 83, 86, 91, 93, and 96 to 102.

In the pharmaceutical composition according to any one of the specific embodiments, the X may include an amino acid sequence selected from the group consisting of SEQ ID NOS: 21, 22, 31, 32, 37, 42, 43, 50, 53, 64, 65, 66, 67, 68, 69, 70, 71, 72, 73, 75, 76, 77, 79, 96, 97, 98, 99, 100, 101, and 102.

In the pharmaceutical composition according to any one of the specific embodiments, the X may include an amino acid sequence selected from the group consisting of SEQ ID NOS: 42, 43, and 50.

In the pharmaceutical composition according to any one of the specific embodiments, the X may be a peptide (essentially) consisting of an amino acid sequence selected from the group consisting of SEQ ID NOS: 1 to 102.

In the pharmaceutical composition according to any one of the specific embodiments, the L is polyethylene glycol having a molecular weight of 1 kDa to 20 kDa.

In the pharmaceutical composition according to any one of the specific embodiments, the non-alcoholic fatty liver disease is selected from the group consisting of non-alcoholic fatty liver (NAFL), non-alcoholic steatohepatitis (NASH), liver fibrosis, cirrhosis, and any combination thereof.

Another aspect of the present invention provides a method of treating a target disease, the method including administering the conjugate or a composition including the same as an active ingredient to an individual in need thereof.

In a specific embodiment, the disease may be obesity and/or a non-alcoholic fatty liver disease.

In the method according to another specific embodiment, the conjugate is parenterally administered to a patient with obesity and/or a non-alcoholic fatty liver disease once a week at dose of 0.5 mg to 8 mg.

In the method according to any one of the specific embodiments, the conjugate or the composition is subcutaneously administered.

Another aspect of the present invention provides a use of the conjugate or a composition including the same as an active ingredient for preventing or treating obesity and/or a non-alcoholic fatty liver disease.

In a specific embodiment, the conjugate is parenterally administered to a patient with obesity and/or a non-alcoholic fatty liver disease once a week at a dose of 0.5 mg to 8 mg.

In the use according to any one of the specific embodiments, the conjugate or the composition is subcutaneously administered.

Another aspect of the present invention provides a preparation including the conjugate as an active ingredient.

In a specific embodiment, the preparation is parenterally administered to a patient with obesity and/or a non-alcoholic fatty liver disease once a week at a dose of 0.5 mg to 8 mg.

In the preparation according to any one of the specific embodiments, the preparation has a use for preventing or treating obesity and/or a non-alcoholic fatty liver disease.

In the preparation according to any one of the specific embodiments, the preparation is subcutaneously administered.

### [Mode for Invention]

Hereinafter, the present invention will be described in detail.

Meanwhile, each of the descriptions and embodiments disclosed herein may be applied to describe different descriptions and embodiments. That is, all of the combinations of various factors disclosed herein belong to the scope of the present invention. Furthermore, the scope of the present invention should not be limited by the detailed descriptions provided hereinbelow.

Throughout the specification, not only the conventional one-letter and three-letter codes for naturally occurring amino acids, but also those three-letter codes generally allowed for other amino acids, such as α-aminoisobutyric acid (Aib), N-methylglycine (Sar), and α-methyl-glutamic acid are used. In addition, the amino acids mentioned herein are abbreviated according to the nomenclature rules of IUPAC-IUB as follows.

| | |
|---|---|
| alanine Ala, A | arginine Arg, R |
| asparagine Asn, N | aspartic acid Asp, D |
| cysteine Cys, C | glutamic acid Glu, E |
| glutamine Gln, Q | glycine Gly, G |
| histidine His, H | isoleucine Ile, I |
| leucine Leu, L | lysine Lys, K |
| methionine Met, M | phenylalanine Phe, F |
| proline Pro, P | serine Ser, S |
| threonine Thr, T | tryptophan Trp, W |
| tyrosine Tyr, Y | valine Val, V |

An aspect of the present invention provides a pharmaceutical composition for preventing or treating obesity and/or a non-alcoholic fatty liver disease, the composition including as an active ingredient a long-acting conjugate of a triple agonist having activities to all of a glucagon receptor, a glucagon-like peptide-1 (GLP-1) receptor, and a glucose-dependent insulinotropic polypeptide (GIP) receptor, wherein the long-acting conjugate of the triple agonist is parenterally administered to a patient with obesity and/or a non-alcoholic fatty liver disease once a week at a dose of 0.5 mg to 8 mg.

The pharmaceutical composition according to the present invention is technically significant in that specific doses and dosing intervals exhibiting safe and effective efficacy have been identified and are actually applicable to the human body.

In a specific aspect of the present invention, the pharmaceutical composition of the present invention may include a triple agonist or a long-acting conjugate thereof as an active ingredient. Specifically, the pharmaceutical composition may include the triple agonist or the long-acting conjugate thereof in a pharmacologically effective amount and a pharmaceutically acceptable excipient, without being limited thereto.

In the present invention, the long-acting conjugate of the triple agonist having activities to a glucagon receptor, a GLP-1 receptor, and a GIP receptor may be in a form in which a peptide (i.e., triple agonist) having activities to the glucagon receptor, the GLP-1 receptor, and the GIP receptor is linked to a biocompatible substance capable of increasing *in vivo* half-life thereof. Throughout the specification, the biocompatible substance may be interchangeably used with carrier.

In the present invention, the long-acting conjugate of the triple agonist may exhibit increased long-lasting effects compared to those of the peptide not linked to the carrier. Meanwhile, the conjugate may be a substance which does not occur naturally.

In a specific embodiment of the present invention, the conjugate is represented by Formula 1 below:

[Formula 1] X-L-F

Here, X is a peptide including one of the amino acid sequences of SEQ ID NOS: 1 to 102;
L is a linker including an ethylene glycol repeating unit;
F is an immunoglobulin Fc region; and
- is a covalent bond between X and L and between L and F.

As used herein, the "peptide having activities to a glucagon receptor, a GLP-1 receptor, and a GIP receptor" may be a component of a moiety constituting the conjugate. Specifically, the peptide corresponds to X in Formula 1 above.

The peptide having activities to glucagon, GLP-1, and GIP receptors may be interchangeably used with triple agonist in the present invention. The triple agonist of the present invention may include a peptide including one of the amino acid sequences of SEQ ID NOS: 1 to 102. Alternatively, the triple agonist of the present invention may include a peptide (essentially) consisting of one of the amino acid sequences of SEQ ID NOS: 1 to 102, without being limited thereto.

The triple agonist or the long-acting conjugate thereof of the present invention may be administered to a patient with obesity and/or a non-alcoholic fatty liver disease in a pharmacologically effective amount.

As used herein, the "pharmacologically effective amount" refers to a safe dose of the triple agonist or a long-acting conjugate thereof having therapeutic effects on the patient with obesity and/or a non-alcoholic fatty liver disease without causing toxicity or side effects in the patient, specifically a dose having effects on losing weight, reducing visceral fat mass, decreasing blood lipid concentration, improving glucose metabolism parameter, reducing blood pressure, and/or reducing fatty liver in the patient with obesity and/or a non-alcoholic fatty liver disease and a dose capable of reducing steatosis grade, number of lobular inflammation, and ballooning degeneration of hepatocytes in liver tissue, resulting in decreases in NAFLD activity score (NAS) and/or fibrosis score. The blood lipid concentration refers to a blood concentration of total cholesterol, LDL-C, HDL-C, VLDL-C, triglyceride, or free fatty acid, and the glucose metabolism parameter may refer to Fasting Plasma Glucose (FPG), Fasting Insulin, Fasting C-Peptide, HbA1c, Homeostatic Model Assessment for Insulin Resistance (HOMA-IR), Homeostatic Model Assessment for Insulin Secretion (HOMA-B), or the like, but is not limited thereto. Additionally, the "pharmacologically effective amount" of the present invention means a dose capable of exhibiting a significant change in a non-alcoholic steatohepatitis (NASH) biomarker (Cytokeratin-18 M30/65 fragments, Enhanced Liver Fibrosis Score, Pro-C3, Non-invasive score 4, Fibrosis-4 index, and NAFLD Fibrosis Score).

Alternatively, the "pharmacologically effective amount" may be a dose capable of exhibiting a maximum concentration (Cₘₐₓ) of 30 ng/mL to 1000 ng/mL after administration or an average area under curve (AUC mean) of 5000 h·ng/mL to 110000 h·ng/mL, without being limited thereto.

The pharmaceutical composition of the present invention may be administered once or more than once so as to maintain the maximum concentration (Cₘₐₓ) or the average area under curve (AUC mean) at a constant level or higher, but is not limited thereto. Specifically, the pharmaceutical composition may be administered to obtain a Cₘₐₓ of 30 ng/mL to 1000 ng/mL, 40 ng/mL to 900 ng/mL, or 41.8 ng/mL to 820 ng/mL, or an average AUC of 5000 h·ng/mL to 110000 h·ng/mL, 5500 h·ng/mL to 105000 h·ng/mL, or 5609.2 h·ng/mL to 100933.9 h·ng/mL in a range of 0.01 mg/kg to 0.12 mg/kg, but is not limited thereto.

In a specific embodiment of the present invention, the long-acting conjugate of the triple agonist according to the present invention may be administered at a dose of about 0.01 mg, 0.02 mg, 0.03 mg, 0.04 mg, 0.05 mg, 0.06 mg, 0.07 mg, 0.08 mg, 0.09 mg, 0.1 mg, 0.2 mg, 0.3 mg, 0.4 mg, 0.5 mg, 0.6 mg, 0.7 mg, 0.8 mg, 0.9 mg, 1 mg, 1.1 mg, 1.2 mg, 1.3 mg, 1.4 mg, 1.5 mg, 1.6 mg, 1.7 mg, 1.8 mg, 1.9 mg, or 2.0 mg or more and about 10 mg, 9.9 mg, 9.8 mg, 9.7 mg, 9.6 mg, 9.5 mg, 9.4 mg, 9.3 mg, 9.2 mg, 9.1 mg, 9 mg, 8.9 mg, 8.8 mg, 8.7 mg, 8.6 mg, 8.5 mg, 8.4 mg, 8.3 mg, 8.2 mg, 8.1 mg, 8 mg, 7.9 mg, 7.8 mg, 7.7 mg, 7.6 mg, 7.5 mg, 7.4 mg, 7.3 mg, 7.2 mg, 7.1 mg, 7 mg, 6.9 mg, 6.8 mg, 6.7 mg, 6.6 mg, 6.5 mg, 6.4 mg, 6.3 mg, 6.2 mg, 6.1 mg, or 6.0 mg or less, without being limited thereto. Specifically, a dose of the long-acting conjugate of the triple agonist of the present invention may be from about 0.01 mg to 10 mg, from about 0.1 mg to 10 mg, from about 0.5 mg to 8 mg, from about 1 mg to 7 mg, or from about 2 mg to 6 mg, but is not limited thereto, and may be appropriately adjusted according to severity of a disease, age of a patient, administration duration, and the like according to the judgement of doctors or prescribers. The dose refers to a dosage that should be administered at regular intervals for treatment of obesity or a non-alcoholic fatty liver disease.

Although the long-acting conjugate of the triple agonist may be administered at a constant dose regardless of a body weight of the patient, if required, the dose may be appropriately adjusted in accordance with the body weight.

As used herein, the term "about" refers to a range including all of ±0.5, ±0.4, ±0.3, ±0.2, ±0.1, ±0.05, ±0.01, or the like and includes all values equivalent to those coming immediately after the term "about" or those in a similar range, without being limited thereto.

The triple agonist or the long-acting conjugate thereof of the present invention may have preventive or therapeutic effects on obesity and/or a non-alcoholic fatty liver disease when administered at a dose at regular intervals for a certain period, without being limited thereto.

The pharmaceutical composition of the present invention may include the triple agonist or the long-acting conjugate in a dose described above or may further include a pharmaceutically acceptable excipient in a required amount, without being limited thereto.

Since the triple agonist is linked to the immunoglobulin Fc region in the long-acting conjugate of the triple agonist of the present invention, the long-acting conjugate of the triple agonist has an increased half-life with the activity of the triple agonist maintained so that pharmacological effects thereof are maintained for a sufficient period of time and dosing intervals may be extended, thereby improving patients' convenience.

Specifically, the long-acting conjugate may be administered once a week, once every two weeks, once every three weeks, once every four weeks, or once a month, but the dosing interval is not limited thereto as long as an *in vivo* concentration capable of providing pharmacological effects thereof is maintained.

The long-acting conjugate of the triple agonist of the present invention may be administered to a patient by way of a fractionated treatment protocol in multiple doses for a long time.

Specifically, the pharmaceutical composition of the present invention may be administered for a period sufficient to obtain a therapeutic effect. For example, the sufficient period may be a period during which one or more abnormal indicators, such as weight, BMI, liver fat content, visceral fat mass, blood lipid concentration, and glucose metabolism parameter of a patient with obesity and/or a non-alcoholic fatty liver disease may return to a range recognized as normal in the art (e.g., BMI less than 23 and liver fat content of 5% or less) or a period during which an indicator used for diagnosis and treatment of a non-alcoholic fatty liver disease in the art, such as NAFLD activity score (NAS), ballooning degeneration of hepatocytes, the number of lobular inflammation, or fibrosis score may return to a normal range. However, the sufficient period may be appropriately determined according to judgement of doctors or prescribers, without being limited thereto. For example, the pharmaceutical composition may be administered for 4 weeks, 5 weeks, 6 weeks, 7 weeks, 8 weeks, 9 weeks, 10 weeks, 11 weeks, 12 weeks, 13 weeks, 14 weeks, 15 weeks, 16 weeks, 17 weeks, 18 weeks, 19 weeks, 20 weeks, 21 weeks, 22 weeks, 23 weeks, 24 weeks, 25 weeks, 26 weeks, 27 weeks, 28 weeks, 29 weeks, 30 weeks, 31 weeks, 32 weeks, 33 weeks, 34 weeks, 35 weeks, 36 weeks, 37 weeks, 38 weeks, 39 weeks, 40 weeks, 41 weeks, 42 weeks, 43 weeks, 44 weeks, 45 weeks, 46 weeks, 47 weeks, 48 weeks, 49 weeks, 50 weeks, 51 weeks, or 52 weeks or more, but the administration period may be appropriately adjusted according to severity of the disease.

The pharmaceutical composition of the present invention may be administered once a week during the above-described administration period.

The pharmaceutical composition of the present invention may be administered to a patient with obesity and/or a non-alcoholic fatty liver disease.

As used herein, the term "obesity" refers to a condition in which adipose tissue is excessive in the body and is generally caused by energy imbalance when an excess energy intake over energy expenditure continues for a long time. Obesity, as a metabolic disease affecting the whole body, may increase the likelihood of getting diabetes and hyperlipidemia, increases the risk of developing sexual dysfunction, arthritis, and cardiovascular disease, and is related to occurrence of cancer.

The pharmaceutical composition of the present invention may be administered to patients with obesity, and a subject patient with obesity may generally be determined based on body mass index (BMI).

In the present invention, the BMI refers to a value obtained by dividing body weight (kg) by the square of height (m), and a patient having a BMI of 23 kg/m² or more, 24 kg/m² or more, 25 kg/m² or more, 26 kg/m² or more, 27 kg/m² or more, 28 kg/m² or more, 29 kg/m² or more, 30 kg/m² or more, 31 kg/m² or more, 32 kg/m² or more, 33 kg/m² or more, 34 kg/m² or more, 35 kg/m² or more, 36 kg/m² or more, 37 kg/m² or more, 38 kg/m² or more, 39 kg/m² or more, or 40 kg/m² or more may be defined as the patient with obesity.

Alternatively, men having a body fat percentage of 25% or more and women having a body fat percentage of 30% or more both measured by bioelectrical impedance analysis, men having a waist size of 90 cm or more and women having a waist size of 85 cm or more, and those having a visceral fat-to-subcutaneous fat ratio of 0.4 or more measured by computed tomography (CT) are diagnosed as patients with obesity. However, patients diagnosed to have obesity according to diagnostic criteria for obesity well known in the art may be administered with the pharmaceutical composition of the present invention.

The pharmaceutical composition of the present invention may have effects on losing weight, reducing visceral fat mass, decreasing blood pressure, and the like in patients with obesity.

As used herein, the term "non-alcoholic fatty liver disease" refers to a type of disease exhibiting histological findings similar to those of alcoholic hepatitis even when the disease is not related to alcohol intake, and the non-alcoholic fatty liver disease may include non-alcoholic fatty liver (NAFL), non-alcoholic steatohepatitis (NASH), liver fibrosis, and cirrhosis. Specifically, the non-alcoholic fatty liver disease of the present invention may include non-alcoholic fatty liver and non-alcoholic steatohepatitis accompanying the same.

The pharmaceutical composition of the present invention may be administered to a patient with a non-alcoholic fatty liver disease, and a subject patient with a non-alcoholic fatty liver disease may generally be determined according to a liver fat content measured by magnetic resonance imaging-derived proton density fat fraction (MRI-PDFF).

In the present invention, the magnetic resonance imaging-derived proton density fat fraction (MRI-PDFF) is an indicator of quantitatively measuring a liver fat content in the liver using MRI, and a patient with a liver fat content of 8% or more, 9% or more, 10% or more, 11% or more, 12% or more, 13% or more, 14% or more, 15% or more, 16% or more, 17% or more, 18% or more, 19% or more, 20% or more, 21% or more, 22% or more, 23% or more, 24% or more, 25% or more, 26% or more, 27% or more, 28% or more, 29% or more, or 30% or more may be determined as the patient with a non-alcoholic fatty liver disease of the present invention.

Alternatively, the patient with a non-alcoholic fatty liver disease may be diagnosed using biochemical methods or imaging methods well known in the art. For example, a patient having an NAFLD activity score (NAS) of 4 or more, an AST/ALT ratio of 1 or more, a controlled attenuation parameter (CAP) value, obtained by a fatty liver measuring technique using a FibroScan device, of 300 dB/m or more, or a fibrosis score of 1 or more may be diagnosed as a patient with a non-alcoholic fatty liver disease. However, patients diagnosed to have a non-alcoholic fatty liver disease according to diagnostic criteria for non-alcoholic fatty liver diseases well known in the art may be administered with the pharmaceutical composition of the present invention.

In the present invention, the patient with a non-alcoholic fatty liver disease may be a patient only with a specific non-alcoholic fatty liver disease but may also be a patient with another disease (e.g., obesity) or multiple non-alcoholic fatty liver diseases. For example, the patient may have both obesity and a non-alcoholic fatty liver disease (e.g., non-alcoholic steatohepatitis (NASH)) or both non-alcoholic fatty liver (NAFL) and non-alcoholic steatohepatitis, but the patient is not limited thereto as long as preventive or therapeutic effects may be obtained by administering the pharmaceutical composition of the present invention.

The pharmaceutical composition of the present invention may have pharmacological effects on preventing or treating a non-alcoholic fatty liver disease, such as effects on losing weight, reducing visceral fat mass, reducing liver fat content, decreasing blood lipid concentration, and improving glucose metabolism parameter, in a patient with a non-alcoholic fatty liver disease. Accordingly, the pharmaceutical composition may have preventive or therapeutic effects on a non-alcoholic fatty liver disease by reducing steatosis grade, decreasing NAFLD activity score (NAS), reducing ballooning degeneration of hepatocytes or the number of lobular inflammation, decreasing fibrosis score, and/or significantly changing a biomarker of NASH (Cytokeratin-18 M30/65 fragments, Enhanced Liver Fibrosis Score, Pro-C3, Non-invasive score 4, Fibrosis-4 index, or NAFLD Fibrosis Score) in liver tissue.

A dose of the pharmaceutical composition of the present invention may be adjusted according to severity of obesity and/or the non-alcoholic fatty liver disease and administration duration.

The pharmaceutical composition of the present invention may be formulated into various dosage forms in combination with a pharmaceutically acceptable excipient. For example, the pharmaceutical composition of the present invention may be formulated into an appropriate dosage form for administration, particularly parenteral administration, of the triple agonist. In addition, the pharmaceutical composition of the present invention may be formulated into a stable dosage form capable of maintaining pharmacological activity of the triple agonist or the long-acting conjugate thereof, without being limited thereto.

For example, the pharmaceutical composition of the present invention may be formulated into a unit dosage form of an ampule or a multidose form. The pharmaceutical composition may also be formulated into solutions, suspensions, tablets, pills, capsules, sustained-release preparations, and the like.

Specifically, the pharmaceutical composition may be formulated into an injectable preparation for subcutaneous administration.

The injectable preparation is directly administered into the human body via a subcutaneous route, a blood vessel (intravascular injection), or a muscle (intramuscular injection) without undergoing absorption in the gastrointestinal tract or metabolism in the liver, and thus effects thereof may be directly obtained regardless of solubility or bioavailability thereof. However, it is necessary to be considerably careful about toxicity caused by a drug component which may directly affect tissue of the human body. That is, stability of the parenteral route may be ensured based only on clinical trial designs and results thereof obtained in consideration of pharmacological effects, side effects, and tolerated doses (dose for safe administration).

Also, the pharmaceutical composition of the present invention may be formulated in a unit dosage form suitable for administration to the body of a patient, specifically in a form useful for administration of protein medicines, according to a method commonly used in the pharmaceutical field and administered via a parenteral administration route commonly used in the art, and one of ordinary skill in the art may appropriately use an intradermal, intravenous, intramuscular, intraarterial, intramedullary, intrathecal, intraventricular, intrapulmonary, transdermal, subcutaneous, intraperitoneal, intranasal, intestinal, topical, sublingual, vaginal, or rectal route as the parenteral administration route. Specifically, the pharmaceutical composition of the present invention may be administered via subcutaneous administration. More specifically, the pharmaceutical composition of the present invention may be administered to an arm (upper arm), thigh, or abdomen of the patient via subcutaneous administration, without being limited thereto.

In view of the objects of the present invention, the pharmaceutical composition may have preventive or therapeutic effects on obesity and/or a non-alcoholic fatty liver disease by exhibiting at least one of properties (a) to (f) below in an individual administered with the pharmaceutical composition:
(a) weight loss;
(b) blood pressure decrease;
(c) visceral fat mass reduction in the liver;
(d) NAS decrease;
(e) reduction in ballooning degeneration of hepatocytes or the number of lobular inflammation; and
(f) fibrosis score decrease.

Specifically, in the patient administered with the pharmaceutical composition of the present invention, a fat mass may be decreased by 30% or more and a weight may be decreased by 5% or more when compared with those in the initial stage of administration, but the embodiment is not limited thereto.

The pharmaceutical composition of the present invention may include a peptide (triple agonist) including or (essentially) consisting of one of the amino acid sequences of SEQ ID NOS: 1 to 102 in a pharmacologically effective amount, without being limited thereto.

In the present invention, the triple agonist may be in the form of a long-acting conjugate represented by Formula 1 below:

[Formula 1] X-L-F

Here,
X is a peptide including one of the amino acid sequences of SEQ ID NOS: 1 to 102;
L is a linker including an ethylene glycol repeating unit;
F is an immunoglobulin Fc region; and
- is a covalent bond between X and L and between L and F.

In the conjugate, X corresponds to the triple agonist of the present invention, specifically a peptide including or (essentially) consisting of one of the amino acid sequences of SEQ ID NOS: 1 to 102.

Although described as a peptide "consisting of" a particular SEQ ID NO: in the present invention, it does not exclude a mutation that may occur naturally or by addition of a meaningless sequence upstream or downstream of the amino acid sequence of the SEQ ID NO, or a silent mutation thereof, as long as the peptide has activity identical or equivalent to that of the peptide consisting of the amino acid sequence, and even when such sequence addition or mutation is present, it obviously belongs to the scope of the present invention. That is, any partial difference in the sequence may fall within the scope of the present invention as long as the peptides have a homology above a certain level and having activity to the glucagon receptor.

For example, for the triple agonists of the present invention, refer to WO 2017-116204 and WO 2017-116205.

Although not particularly limited, the triple agonist having significant levels of activities to the glucagon, GLP-1, and GIP receptors may exhibit *in vitro* activities of about 0.1% or more, 1% or more, 2% or more, 3% or more, 4% or more, 5% or more, 6% or more, 7% or more, 8% or more, 9% or more, 10% or more, 20% or more, 30% or more, 40% or more, 50% or more, 60% or more, 70% or more, 80% or more, 90% or more, or 100% or more, to at least one receptor, specifically to at least two receptors, and more specifically to all three of the glucagon, GLP-1, and GIP receptors, compared to native ligands of the corresponding receptors (native glucagon, native GLP-1, and native GIP).

For a method for measuring the *in vitro* activities of the triple agonist, refer to Experimental Example 1 of the present invention, without being limited thereto.

Meanwhile, the peptide is characterized by having one or more, specifically three, of the following activities of i) to iii), specifically a significant activity(ies) thereof:
i) activation of a GLP-1 receptor; ii) activation of a glucagon receptor; and iii) activation of a GIP receptor.

In this regard, the activation of the receptor may include, for example, cases where the *in vitro* activities are 0.1% or more, 1% or more, 2% or more, 3% or more, 4% or more, 5% or more, 6% or more, 7% or more, 8% or more, 9% or more, 10% or more, 20% or more, 30% or more, 40% or more, 50% or more, 60% or more, 70% or more, 80% or more, 90% or more, or 100% or more, compared to native forms, but the embodiment is not limited thereto.

In addition, the peptide may be one having an increased *in vivo* half-life relative to any one of native GLP-1, native glucagon, and native GIP, without being limited thereto.

Although not particularly limited, the peptide may be one which is not naturally occurring.

Even in the form of the conjugate of the present invention, significant activities to the glucagon receptor, the GLP-1 receptor, and the GIP receptor may be obtained, and thus the conjugate may exert preventive or therapeutic effects on obesity and/or a non-alcoholic fatty liver disease.

Specifically, the conjugate of the present invention may have *in vitro* activities of 0.01% or more, 0.1% or more, 0.2% or more, 0.5% or more, 0.7% or more, 1% or more, 2% or more, 3% or more, 4% or more, 5% or more, 6% or more, 7% or more, 8% or more, 9% or more, 10% or more, 20% or more, 30% or more, 40% or more, 50% or more, 60% or more, 70% or more, 80% or more, 90% or more, 100% or more to the glucagon receptor, the GLP-1 receptor, and/or the GIP receptor compared to native forms thereof, without being limited thereto.

In a specific aspect of the present invention, the triple agonist may include one of the amino acid sequences of SEQ ID NOS: 1 to 102, (essentially) consist of one of the amino acid sequences of SEQ ID NOS: 1 to 102, or include a peptide having a sequence identity of 60% or more, 65% or more, 70% or more, 75% or more, 80% or more, 85% or more, 90% or more, 91% or more, 92% or more, 93% or more, 94% or more, or 95% or more with one of the amino acid sequences of SEQ ID NOS: 1 to 102, but is not particularly limited thereto as long as the peptide have therapeutic effects on obesity or the non-alcoholic fatty liver disease.

In another specific aspect, the triple agonist may include one of the amino acid sequences of SEQ ID NOS: 21, 22, 27, 30 to 32, 34, 36, 37, 42, 43, 50 to 56, 58, 64 to 80, 83, 86, 91, 93, and 96 to 102, (essentially) consist of one of the amino acid sequences of SEQ ID NOS: 21, 22, 27, 30 to 32, 34, 36, 37, 42, 43, 50 to 56, 58, 64 to 80, 83, 86, 91, 93, and 96 to 102, or include a peptide having a sequence identity of 60% or more, 65% or more, 70% or more, 75% or more, 80% or more, 85% or more, 90% or more, 91% or more, 92% or more, 93% or more, 94% or more, or 95% or more with one of the amino acid sequences of SEQ ID NOS: 21, 22, 27, 30 to 32, 34, 36, 37, 42, 43, 50 to 56, 58, 64 to 80, 83, 86, 91, 93, and 96 to 102, without being limited thereto.

In another specific aspect, the triple agonist may include one of the amino acid sequences of SEQ ID NOS: 21, 22, 31, 32, 37, 42, 43, 50, 53, 64, 65, 66, 67, 68, 69, 70, 71, 72, 73, 75, 76, 77, 79, 96, 97, 98, 99, 100, 101, and 102, (essentially) consist of one of the amino acid sequences of SEQ ID NOS: 21, 22, 31, 32, 37, 42, 43, 50, 53, 64, 65, 66, 67, 68, 69, 70, 71, 72, 73, 75, 76, 77, 79, 96, 97, 98, 99, 100, 101, and 102, or include a peptide having a sequence identity of 60% or more, 65% or more, 70% or more, 75% or more, 80% or more, 85% or more, 90% or more, 91% or more, 92% or more, 93% or more, 94% or more, or 95% or more with one of the amino acid sequences of SEQ ID NOS: 21, 22, 31, 32, 37, 42, 43, 50, 53, 64, 65, 66, 67, 68, 69, 70, 71, 72, 73, 75, 76, 77, 79, 96, 97, 98, 99, 100, 101, and 102, without being limited thereto.

In another specific aspect, the triple agonist may include one of the amino acid sequences of SEQ ID NOS: 42, 43 and 50, (essentially) consist of one of the amino acid sequences of SEQ ID NOS: 42, 43 and 50, or include a peptide having a sequence identity of 60% or more, 65% or more, 70% or more, 75% or more, 80% or more, 85% or more, 90% or more, 91% or more, 92% or more, 93% or more, 94% or more, or 95% or more with one of the amino acid sequences of SEQ ID NOS: 42, 43 and 50, without being limited thereto.

As used herein, the term "homology" or "identity" refers to the degree of relatedness between two given amino acid sequences or nucleotide sequences and may be expressed as a percentage.

The terms homology and identity may often be used interchangeably.

The sequence homology, similarity, or identity between two given peptide sequences may be determined using any known computer algorism such as "FASTA" program by using default parameters as introduced by, for example, Pearson et al (1988) Proc. Natl. Acad. Sci. USA 85:2444. Alternatively, the Needleman-Wunsch algorithm (1970, J. Mol. Biol. 48:443-453) performed in a Needleman program of The European Molecular Biology Open Software Suite (EMBOSS) package (Rice et al., 2000, Trends Genet. 16:276-277) (version 5.0.0 or later) may be used to determine the same (including GCG program package (Devereux, J. et al., Nucleic Acids Research 12:387 (1984)), BLASTP, BLASTN, FASTA (Atschul, S. F. et al., J MOLEC BIOL 215:403 (1990); Guide to Huge Computers, Martin J. Bishop, ed., Academic Press, San Diego,1994, and CARILLO et al. (1988) SIAM J Applied Math 48:1073). For example, the homology, similarity, or identity may be determined using BLAST from The National Center for Biotechnology Information database, or ClustalW.

The homology, similarity, or identity between polypeptides may be determined by comparing sequence information using a GAP computer program, such as a program introduced by Needleman et al. (1970), J Mol Biol. 48:443 as disclosed in Smith and Waterman, Adv. Appl. Math (1981) 2:482. In brief, the GAP program defines similarity as the number of aligned symbols (i.e., amino acids) which are similar, divided by the total number of symbols in a shorter of two sequences. Default parameters for the GAP program may include: (1) a binary comparison matrix (containing a value of 1 for identity and 0 for non-identity) a weighted comparison matrix of Gribskov et al. (1986) Nucl. Acids Res. 14:6745 disclosed in Schwartz and Dayhoff, eds., Atlas Of Protein Sequence And Structure, National Biomedical Research Foundation, pp. 353-358 (1979) (or EDNAFULL (EMBOSS version of NCBI NUC4.4) substitution matrix); (2) a penalty of 3.0 for each gap and an additional 0.10 penalty for each symbol in each gap (or gap open penalty of 10, and a gap extension penalty of 0.5); and (3) no penalty for end gaps. Therefore, as used herein, the term "homology" or "identity" indicates relatedness between sequences.

The triple agonist may include an intramolecular bridge (*e.g*., covalent crosslinking or non-covalent crosslinking), and specifically, it is in a form including a ring, for example, is in a form where a ring is formed between the 16^{th} and 20^{th} amino acids of the triple agonist, without being limited thereto.

Non-limiting examples of the ring may include a lactam bridge (or a lactam ring).

In addition, the triple agonist includes all of those modified to include a ring or include an amino acid capable of forming a ring at a target position.

For example, the triple agonist may be one where a pair of 16^{th} and 20^{th} amino acids are substituted with glutamic acid or lysine capable of forming a ring, but is not limited thereto.

The ring may be formed between side chains of amino acids in the triple agonist, *e.g*., a lactam ring may be formed between a side chain of lysine and a side chain of glutamic acid, but is not limited thereto.

Also, although not particularly limited, in the peptide of the present invention, some amino acids may be substituted with different amino acids or a non-native compound to avoid recognition by a degradation enzyme for increasing *in vivo* half-life.

Specifically, the peptide may be one having an increased *in vivo* half-life by avoiding recognition by the degradation enzyme via substitution of the 2^{nd} amino acid in the amino acid sequence of the triple agonist, but any substitution or modification of amino acids to avoid recognition by the degradation enzyme in living organisms may be used without limitation.

In addition, such modification for the preparation of the triple agonist includes modification using L-type or D-type amino acids and/or non-native amino acids; and/or modification of a native sequence, such as modification of a side-chain functional group, intramolecular covalent bond, such as ring formation between side chains, methylation, acylation, ubiquitination, phosphorylation, aminohexanation, and biotinylation.

Also, the modification includes all cases where one or more amino acids are added to the amino and/or carboxy terminal of the triple agonist.

The amino acids substituted or added may be not only 20 amino acids commonly found in human proteins but also atypical amino acids or those which do not occur naturally. Commercial sources of atypical amino acids may include Sigma-Aldrich, ChemPep Inc. and Genzyme pharmaceuticals. The peptides including these amino acids and typical peptide sequences may be synthesized and purchased from commercial peptide suppliers, e.g., American Peptide Company, Bachem, or Anygen (Korea).

Amino acid derivatives may also be obtained in the same manner; for example, 4-imidazoacetic acid may be used.

In addition, the triple agonist according to the present invention may be in a varied form where the N-terminus and/or C-terminus is chemically modified or protected by organic groups or amino acids may be added to the termini of the peptide, for protection from proteases in living organisms while increasing stability thereof.

Particularly, since the N- and C-termini of chemically synthesized peptides are electrically charged, the N-terminus may be acetylated and/or the C-terminus may be amidated to remove the charges, but the present invention is not limited thereto.

In addition, the triple agonist according to the present invention includes all of those in the form of the triple agonist itself, a salt thereof (*e.g.*, a pharmaceutically acceptable salt of the peptide), or a solvate thereof. Also, the peptide may be in any pharmaceutically acceptable form.

The type of the salt is not particularly limited. However, the salt is preferably in a form safe and effective to an individual, *e.g*., a mammal, without being limited thereto.

The term "pharmaceutically acceptable" refers to a substance that may be effectively used for the intended use within the scope of pharmaco-medical decision without inducing excessive toxicity, irritation, allergic responses, and the like.

As used herein, the term "pharmaceutically acceptable salt" refers to a salt derived from a pharmaceutically acceptable inorganic acid, organic acid, or base. Examples of a suitable acid may include hydrochloric acid, bromic acid, sulfuric acid, nitric acid, perchloric acid, fumaric acid, maleic acid, phosphoric acid, glycolic acid, lactic acid, salicylic acid, succinic acid, toluene-p-sulfonic acid, tartaric acid, acetic acid, citric acid, methanesulfonic acid, formic acid, benzoic acid, malonic acid, naphthalene-2-sulfonic acid, and benzenesulfonic acid. Examples of the salt derived from a suitable base may include alkali metals such as sodium and potassium, alkali earth metals such as magnesium and ammonium.

In addition, as used herein, the term "solvate" refers to a complex of the triple agonist or a salt thereof according to the present invention and a solvent molecule.

Meanwhile, the peptide of the present invention may be synthesized, according to the length, by a method well known in the art, e.g., by an automatic peptide synthesizer, and may be produced by genetic engineering technology.

Specifically, the peptide of the present invention may be prepared by a standard synthesis method, a recombinant expression system, or any other method known in the art. Thus, the peptide according to the present invention may be synthesized by a plurality of methods including the following methods:
(a) a method of synthesizing a peptide in a stepwise or fragment assembling manner by a solid-phase or liquid-phase method, followed by isolation and purification of a final peptide product;
(b) a method of expressing a nucleic acid construct encoding a peptide in a host cell and recovering an expressed product from a host cell culture;
(c) a method of performing *in vitro* cell-free expression of a nucleic acid construct encoding a peptide and recovering an expressed product therefrom; or
a method of obtaining peptide fragments by any combination of the methods (a), (b), and (c), obtaining the peptide by linking the peptide fragments, and then recovering the peptide.

In the long-acting conjugate of Formula 1 above, F is a substance capable of increasing the half-life of X, i.e., the peptide with activities to the glucagon receptor, the GLP-1 receptor, and the GIP receptor, corresponding to a component of a moiety constituting the conjugate of the present invention.

F and X may be linked to each other by a covalent chemical bond or a non-covalent chemical bond, and F and X may be linked to each other via L by a covalent chemical bond, a non-covalent chemical bond, or any combination thereof.

More specifically, X and L and L and F may be linked to each other via covalent bonds, and the conjugate is a conjugate in which X, L, and F are linked via covalent bonds in the order as shown in Formula 1.

The F may be the immunoglobulin Fc region, and more specifically, the immunoglobulin Fc region may be derived from IgG, without being limited thereto.

As used herein, the term "immunoglobulin Fc region" refers to a region including a heavy chain constant domain 2 (CH2) and/or a heavy chain constant domain 3 (CH3) excluding the heavy chain and light chain variable domains of the immunoglobulin. The immunoglobulin Fc region may be a component constituting a moiety of the conjugate of the present invention.

Throughout the specification, the Fc region includes not only a native sequence obtained from papain digestion of immunoglobulin but also derivatives thereof, e.g., a sequence different from the native sequence and obtained by modification of one or more amino acid residues via deletion, addition, non-conservative or conservative substitution, or any combination thereof.

F has a structure in which two polypeptide chains are linked to each other by a disulfide bond, wherein the linkage is formed by a nitrogen atom of one of the two chains, but is not limited thereto. Linkage via the nitrogen atom may be formed by reductive amination of an ε-amino group or an N-terminal amino group of lysine.

The reductive amination refers to a reaction in which an amine group or an amino group of one reactant reacts with an aldehyde group (a functional group capable of participating reactive amination) of another reactor to produce an amine, and then an amine bond is formed by reduction, as an organic synthesis reaction well known in the art.

In a specific embodiment, F may be linked via the nitrogen atom of the N-terminal proline, without being limited thereto.

The immunoglobulin Fc region, as a component constituting a moiety of the conjugate of Formula 1 of the present invention, may correspond, specifically to F in Formula 1 shown above.

The immunoglobulin Fc region may include a hinge region in the heavy chain constant domain, but is not limited thereto.

In the present invention, the immunoglobulin Fc region may include a particular hinge sequence at the N-terminus.

As used herein, the term "hinge sequence" refers to a site located at a heavy chain and forming a dimer of the immunoglobulin Fc region via an inter disulfide bond.

In the present invention, the hinge sequence may be mutated to have one cysteine residue by deletion of a part of the hinge sequence including the flowing amino acid sequence, but is not limited thereto.

Glu-Ser-Lys-Tyr-Gly-Pro-Pro-Cys-Pro-Ser-Cys-Pro (SEQ ID NO: 103).

The hinge sequence may include one cysteine residue since the cysteine residue at position 8 or 11 is deleted from the hinge sequence of SEQ ID NO: 103. The hinge sequence of the present invention may consist of 3 to 12 amino acids including only one cysteine residue, but is not limited thereto. More specifically, the hinge sequence of the present invention may have a sequence as follows: Glu-Ser-Lys-Tyr-Gly-Pro-Pro-Pro-Ser-Cys-Pro (SEQ ID NO: 104), Glu-Ser-Lys-Tyr-Gly-Pro-Pro-Cys-Pro-Ser-Pro (SEQ ID NO: 105), Glu-Ser-Lys-Tyr-Gly-Pro-Pro-Cys-Pro-Ser (SEQ ID NO: 106), Glu-Ser-Lys-Tyr-Gly-Pro-Pro-Cys-Pro-Pro (SEQ ID NO: 107), Lys-Tyr-Gly-Pro-Pro-Cys-Pro-Ser (SEQ ID NO: 108), Glu-Ser-Lys-Tyr-Gly-Pro-Pro-Cys (SEQ ID NO: 109), Glu-Lys-Tyr-Gly-Pro-Pro-Cys (SEQ ID NO: 110), Glu-Ser-Pro-Ser-Cys-Pro (SEQ ID NO: 111), Glu-Pro-Ser-Cys-Pro (SEQ ID NO: 112), Pro-Ser-Cys-Pro (SEQ ID NO: 113), Glu-Ser-Lys-Tyr-Gly-Pro-Pro-Ser-Cys-Pro (SEQ ID NO: 114), Lys-Tyr-Gly-Pro-Pro-Pro-Ser-Cys-Pro (SEQ ID NO: 115), Glu-Ser-Lys-Tyr-Gly-Pro-Ser-Cys-Pro (SEQ ID NO: 116), Glu-Ser-Lys-Tyr-Gly-Pro-Pro-Cys (SEQ ID NO: 117), Lys-Tyr-Gly-Pro-Pro-Cys-Pro (SEQ ID NO: 118), Glu-Ser-Lys-Pro-Ser-Cys-Pro (SEQ ID NO: 119), Glu-Ser-Pro-Ser-Cys-Pro (SEQ ID NO: 120), Glu-Pro-Ser-Cys (SEQ ID NO: 121), or Ser-Cys-Pro (SEQ ID NO: 122).

More specifically, the hinge sequence may include an amino acid sequence of SEQ ID NO: 113 (Pro-Ser-Cys-Pro) or SEQ ID NO: 122 (Ser-Cys-Pro), but is not limited thereto.

The immunoglobulin Fc region of the present invention may be in dimer form including two chain molecules of the immunoglobulin Fc region in the presence of the hinge sequence, and the conjugate represented by Formula 1 according to the present invention may be in a form in which one end of the linker is linked to one chain of the immunoglobulin Fc region as a dimer, without being limited thereto.

As used herein, the term "N-terminus" refers to an amino terminus of a protein or polypeptide and may include an amino acid residue located at the end of the amino terminus or 1, 2, 3, 4, 5, 6, 7, 8, 9, 10 or more amino acids from the end of the amino terminus. The immunoglobulin Fc region of the present invention may include the hinge sequence at the N-terminus, without being limited thereto.

Also, the immunoglobulin Fc region of the present invention may be an extended Fc region including a part of or the entirety of a heavy chain constant domain 1 (CH1) and/or a light chain constant domain 1 (CL1) excluding the heavy chain and the light chain variable domains of the immunoglobulin, as long as the immunoglobulin Fc region has substantially identical or enhanced effects compared to the native type. Also, the immunoglobulin Fc region may be a region from which a considerably long part of the amino acid sequence corresponding to the CH2 and/or CH3 is removed.

For example, the immunoglobulin Fc region of the present invention may include 1) a CH1 domain, a CH2 domain, a CH3 domain, and a CH4 domain, 2) a CH1 domain and a CH2 domain, 3) a CH1 domain and a aCH3 domain, 4) a CH2 domain and a CH3 domain, 5) a combination of one or more domains selected from a aCH1 domain, a CH2 domain, a CH3 domain, and a CH4 domain and an immunoglobulin hinge region (or a part of the hinge region), or 6) a dimer of each of the heavy chain constant domains and the light chain constant domain, but is not limited thereto.

Also, in the long-acting conjugate according to an embodiment of the present invention, F, the immunoglobulin Fc region, is in the form of a dimer formed of two polypeptide chains and is covalently linked to X via a single linker L including an ethylene glycol repeating unit. In a specific example of the embodiment, X is covalently linked to only one of the two polypeptide chains of the Fc region dimer F via the linker L. In a more specific example of the embodiment, only one X molecule is covalently linked to one of the two polypeptide chains of the Fc region dimer F via the linker L. In a most specific example of the embodiment, F is a homodimer.

In another specific example, F, the immunoglobulin Fc region, is in the form of a dimer formed of two polypeptide chains and one end of L is linked to only one of the two polypeptide chains polypeptide, without being limited thereto.

In the long-acting conjugate according to another embodiment of the present invention, two X molecules may also be symmetrically linked to one Fc region in a dimeric form. In this case, the immunoglobulin Fc and X may be linked to each other by the non-peptidyl linker. However, the embodiment is not limited thereto.

In addition, the immunoglobulin Fc region of the present invention includes not only the native amino acid sequence but also a sequence derivative thereof. The amino acid sequence derivative is a sequence that is different from the native amino acid sequence due to deletion, insertion, non-conservative or conservative substitution of one or more amino acid residues, or any combination thereof.

For example, in an IgG Fc, amino acid residues known to be important in linkage at positions 214 to 238, 297 to 299, 318 to 322, or 327 to 331 may be used as a suitable target for modification.

Also, other various derivatives are possible, including those in which a region capable of forming a disulfide bond is deleted or certain amino acid residues are eliminated at the N-terminus of a native Fc form, and a methionine residue is added thereto may be used. Further, to remove effector functions, a deletion may occur in a complement binding site, such as a C1q binding site and an antibody dependent cell mediated cytotoxicity (ADCC) site. Techniques of preparing such sequence derivatives of the immunoglobulin Fc region are disclosed in International Patent Publication Nos. WO 97/34631 and WO 96/32478.

Amino acid exchanges in proteins and peptides, which do not generally alter the activity of molecules, are known in the art (H. Neurath, R. L. Hill, The Proteins, Academic Press, New York, 1979). The most commonly occurring exchanges are Ala/Ser, Val/Ile, Asp/Glu, Thr/Ser, Ala/Gly, Ala/Thr, Ser/Asn, Ala/Val, Ser/Gly, Thy/Phe, Ala/Pro, Lys/Arg, Asp/Asn, Leu/Ile, Leu/Val, Ala/Glu, and Asp/Gly in both directions. The Fc region, if desired, may be modified by phosphorylation, sulfation, acrylation, glycosylation, methylation, farnesylation, acetylation, amidation, and the like.

The above-described Fc derivatives are derivatives that have a biological activity equivalent to the Fc region of the present invention or improved structural stability against heat, pH, or the like.

In addition, these Fc regions may be obtained from native forms isolated from humans and other animals including cows, goats, swine, mice, rabbits, hamsters, rats and guinea pigs, or may be recombinants or derivatives thereof, obtained from transformed animal cells or microorganisms. In this regard, they may be obtained from a native immunoglobulin by isolating whole immunoglobulins from human or animal organisms and treating them with a proteolytic enzyme. Papain digests the native immunoglobulin into Fab and Fc regions, and pepsin treatment results in the production of pF'c and F(ab)₂ fragments. These fragments may be subjected to size exclusion chromatography to isolate Fc or pF'c. In a more specific example, a human-derived Fc region is a recombinant immunoglobulin Fc region that is obtained from a microorganism.

In addition, the immunoglobulin Fc region may have natural glycans or increased or decreased glycans compared to the natural type, or be in a deglycosylated form. The increase, decrease, or removal of glycans of the immunoglobulin Fc may be achieved by any methods commonly used in the art such as a chemical method, an enzymatic method, and a genetic engineering method using a microorganism. In this regard, the immunoglobulin Fc region obtained by removing glycans shows a significant decrease in binding affinity to a complement c1q and a decrease in or loss of antibody-dependent cytotoxicity or complement-dependent cytotoxicity, and thus unnecessary immune responses are not induced thereby in living organisms. Based thereon, a deglycosylated or aglycosylated immunoglobulin Fc region may be more suitable as a drug carrier in view of the objects of the present invention.

As used herein, the term "deglycosylation" refers to a Fc region from which glycan is removed using an enzyme and the term "aglycosylation" refers to a Fc region that is not glycosylated and produced in prokaryotes, more specifically *E*. *coli.*

Meanwhile, the immunoglobulin Fc region may be derived from humans or animals such as cows, goats, pigs, mice, rabbits, hamsters, rats, or guinea pigs. In a more specific embodiment, the immunoglobulin Fc region may be derived from humans.

In addition, the immunoglobulin Fc region may be derived from IgG, IgA, IgD, IgE, or IgM, or any combination or hybrid thereof. In a more specific embodiment, the immunoglobulin Fc region is derived from IgG or IgM which are the most abundant proteins in human blood, and in an even more specific embodiment, it is derived from IgG known to enhance the half-lives of ligand binding proteins. In a yet even more specific embodiment, the immunoglobulin Fc region is an IgG4 Fc region, and in the most specific embodiment, the immunoglobulin Fc region is an aglycosylated Fc region derived from human IgG4, without being limited thereto.

In addition, in a specific embodiment, the immunoglobulin Fc fragment may be a human IgG4 Fc fragment in the form of a homodimer in which two monomers are linked to each other via a disulfide bond (inter-chain) formed between cysteines that are amino acids located at position 3 of each monomer. In this regard, each monomer of the homodimer has or may have independent two inner disulfide bonds (intra-chain), *i.e*., a disulfide bond formed between cysteines at positions 35 and 95 and a disulfide bond formed between cysteines at positions 141 and 199. Each monomer may consist of 221 amino acids and the number of amino acids constituting the homodimer may be 442 in total, without being limited thereto. Specifically, the immunoglobulin Fc fragment may be in the form of a homodimer in which two monomers each having an amino acid sequence of SEQ ID NO: 123 (consisting of 221 amino acids) are linked to each other via a disulfide bond between cysteines at position 3 of each monomer, wherein the monomers of the homodimer each independently have a disulfide bond formed between cysteines at positions 35 and 95 and a disulfide bond formed between cysteines at positions 141 and 199, without being limited thereto.

Meanwhile, as used herein, the term "combination" related to the immunoglobulin Fc region refers to formation of a linkage between a polypeptide encoding a single-chain immunoglobulin Fc region of the same origin and a single-chain polypeptide of a different origin when a dimer or a multimer is formed. That is, a dimer or multimer may be prepared using two or more Fc fragments selected from the group consisting of IgG Fc, IgA Fc, IgM Fc, IgD Fc, and IgE Fc fragments.

As used herein, the term "hybrid" means that sequences corresponding to two or more immunoglobulin Fc fragments of different origins are present in a single-chain of an immunoglobulin constant domain. In the present invention, various hybrid forms are possible. That is, a domain hybrid may be composed of 1 to 4 domains selected from the group consisting of CH1, CH2, CH3, and CH4 of IgG Fc, IgM Fc, IgA Fc, IgE Fc, and IgD Fc and may further include a hinge region.

Meanwhile, the IgG may also be divided into IgG1, IgG2, IgG3 and IgG4 subclasses, which may be combined or hybridized in the present invention. Preferred are IgG2 and IgG4 subclasses, and most preferred is the Fc fragment of IgG4 rarely having effector functions such as complement dependent cytotoxicity (CDC).

In addition, the above-described conjugate may have an improved long-acting property of the effect compared to the native GLP-1, GIP, or glucagon, or compared to X not modified with F, and the conjugate may include a form enclosed in biodegradable nanoparticles as well as those described above, without being limited thereto.

Meanwhile, L may be a non-peptidyl linker, e.g., a linker including an ethylene glycol repeating unit.

In the present invention, the "non-peptidyl linker" includes a biocompatible polymer in which two or more repeating units are linked. The repeating units are linked to each other via any covalent bond other than a peptide bond. The non-peptidyl linker may be a component constituting a moiety of the conjugate of the present invention and corresponds to L in Formula 1.

As the non-peptidyl linker used in the present invention, any polymer having resistance to proteases in living organisms may be used without limitation. In the present invention, the non-peptidyl linker may be interchangeably used with the non-peptidyl polymer.

In addition, the non-peptidyl linker of the present invention linked to the polypeptide corresponding to F may be not only a polymer of one type but also a combination different types of polymers.

In a specific embodiment, the conjugate may be one in which F and X are covalently linked to each other via the non-peptidyl linker including reactive groups at both ends binding to F, specifically the immunoglobulin Fc region, and the other binding to X, specifically the triple agonist.

Specifically, in the present invention, the non-peptidyl linker may include reactive groups at both ends to form a conjugate another component constituting the conjugate by reaction. In the case where a non-peptidyl linker having reactive functional groups at both ends binds to X and F of Formula 1 via the reactive groups, the non-peptidyl linker or the non-peptidyl polymer may be named a non-peptidyl polymer linker moiety or a non-peptidyl linker linking unit.

Although not particularly limited, the non-peptidyl linker may be a linker including an ethylene glycol repeating unit, *e.g*., polyethylene glycol, and any derivatives thereof well known in the art and easily prepared at the level of the technology in the art belong to the scope of the present invention.

The repeating unit of the non-peptidyl linker may be an ethylene glycol repeating unit, and specifically, the non-peptidyl linker may include an ethylene glycol repeating unit and functional groups used for preparation of the conjugate at both ends thereof. In the long-acting conjugate according to the present invention, X may be linked to F via the functional groups, without being limited thereto. In the present invention, the non-peptidyl linker may include two, three, or more functional groups, which may be the same or different, without being limited thereto.

Specifically, the linker may be polyethylene glycol (PEG) represented by Chemical Formula 2 below, without being limited thereto:

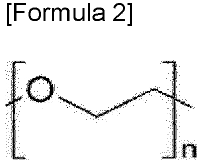

In Formula 2, n may be from 10 to 2400, n may be from 10 to 480, or n may be from 50 to 250, without being limited thereto.

In the long-acting conjugate, the PEG moiety may include not only a - (CH₂CH₂O)ₙ- structure and an oxygen atom interposed between a linking element and the -(CH₂CH₂O)ₙ- structure, without being limited thereto.

Also, in a specific embodiment, the conjugate may have a structure in which the triple agonist (X) is linked to the immunoglobulin Fc region (F) via the linker including an ethylene glycol repeating unit by covalent bonds, without being limited thereto.

The polyethylene glycol is a term including all forms of an ethylene glycol homopolymer, a PEG copolymer, and a monomethyl-substituted PEG polymer (mPEG), without being limited thereto.

The non-peptidyl linker to be used in the present invention may be any polymer including an ethylene glycol repeating unit resistant to proteases in living organisms, without limitation. A molecular weight of the non-peptidyl polymer may be greater than 0 kDa and less than about 100 kDa, in the range of about 1 kDa to about 100 kDa, specifically about 1 kDa to about 20 kDa, or about 1 kDa to about 10 kDa, but is not limited thereto. In addition, the non-peptidyl linker of the present invention linked to a polypeptide corresponding to F may include not only a single type of a polymer but also a combination of different types of polymers.

Specifically, the non-peptidyl linker includes reactive groups at both ends in a state not linked to F and X and is linked to F and X via the reactive groups.

In a specific embodiment, both ends of the non-peptidyl linker may be respectively linked to an amine group or a thiol group of F, *e.g*., the immunoglobulin Fc region, and an amine group or a thiol group of X, respectively.

Specifically, the non-peptidyl polymer may include reactive groups at both ends respectively linked to F (*e.g*., the immunoglobulin Fc region) and X, specifically reactive groups respectively to be linked to an amine group of the N-terminus or lysine or a thiol group of cysteine of X or F (*e.g*., the immunoglobulin Fc region), without being limited thereto.

In addition, the reactive groups to be linked to F, *e.g*., the immunoglobulin Fc region, and X may be selected from the group consisting of an aldehyde group, a maleimide group, and a succinimide derivative, but are not limited thereto.

In the above description, the aldehyde group may be a propionaldehyde group or a butyraldehyde group, without being limited thereto.

In the above description, the succinimide derivative may be succinimidyl valerate, succinimidyl methyl butanoate, succinimidyl methylpropionate, succinimidyl butanoate, succinimidyl propionate, *N*-hydroxysuccinimide, hydroxy succinimidyl, succinimidyl carboxymethyl, or succinimidyl carbonate, without being limited thereto.

The non-peptidyl linker may be linked to X and F via such reactive groups, without being limited thereto.

A final product produced by reductive amination by an aldehyde bond is more stable than that produced by an amide bond. The aldehyde reactive group selectively reacts with the N-terminus at a low pH to form a covalent bond with a lysine residue at a high pH, *e.g*., at a pH of 9.0.

In addition, the reactive groups of both ends of the non-peptidyl linker may be the same or different, for example, a maleimide group may be provided at one end and an aldehyde group, a propionaldehyde group, or a butyraldehyde group may be provided at the other end. However, the reactive groups are not particularly limited as long as F, specifically the immunoglobulin Fc region, and X may be linked to the respective ends of the non-peptidyl linker.

For example, the non-peptidyl linker may include a maleimide group at one end and an aldehyde group, a propionaldehyde group, or a butyraldehyde group at the other end, as reactive groups.

When polyethylene glycol having hydroxyl groups as reactive groups at both ends is used as the non-peptidyl polymer, the long-acting protein conjugate according to the present invention may be prepared by activating the hydroxyl groups to various reactive groups by known chemical reactions, or using commercially available polyethylene glycol having modified reactive groups.

In a specific embodiment, the non-peptidyl polymer may be linked to a cysteine residue, more specifically a -SH group of cysteine, without being limited thereto.

For example, the non-peptidyl polymer may be linked to a cysteine residue located at the 10^{th} position, a cysteine residue located at the 13^{th} position, a cysteine residue located at the 15^{th} position, a cysteine residue located at the 17^{th} position, a cysteine residue located at the 19^{th} position, a cysteine residue located at the 21^{st} position, a cysteine residue located at the 24^{th} position, a cysteine residue located at the 28^{th} position, a cysteine residue located at the 29^{th} position, a cysteine residue located at the 30^{th} position, a cysteine residue located at the 31^{st} position, a cysteine residue located at the 40^{th} position, or a cysteine residue located at the 41^{st} position of the peptide corresponding to X, without being limited thereto. Specifically, a reactive group of the non-peptidyl polymer may be linked to the -SH group of the cysteine residue, and the reactive group is as described above.

When maleimide-PEG-aldehyde is used, the maleimide group may be linked to the -SH group of X via a thioether bond, and the aldehyde group may be linked to the -NH₂ group of F, specifically the immunoglobulin Fc region, via reductive amination, but this is merely an example, and the present invention is not limited thereto.

An oxygen atom located at one end of the PEG is linked to an N-terminal amino group of the immunoglobulin Fc region via a linker functional group having a -CH₂CH₂CH₂- structure via the reductive alkylation to form a -PEG-O-CH₂CH₂CH₂NH-immunoglobulin Fc structure. The other end of the PEG may be linked to a sulfur atom located at cysteine of the triple agonist via the thioether bond. The above-described thioether bond may have a 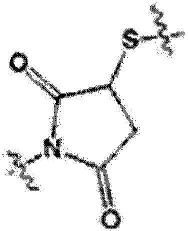 structure.

However, the embodiment is not limited to those described above but is merely an example.

In another specific embodiment, the non-peptidyl polymer may be linked to a lysine residue, more specifically an amino group of lysine, of X, without being limited thereto.

In addition, in the conjugate, the reactive group of the non-peptidyl polymer may be linked to the -NH₂ group located at the N-terminus of the immunoglobulin Fc region, but this is merely an example,

Unless otherwise stated, detailed descriptions about "peptide" of the present invention or the "conjugate" in which the peptide is covalently linked to a biocompatible material, disclosed in the specification or techniques of the claims may be applied not only to the peptide or conjugate but also a salt of the peptide or conjugate (e.g., a pharmaceutically acceptable salt of the peptide), or a solvate form thereof. Thus, although only "peptide" or "conjugate" is described in the specification, descriptions thereof may also be applied to particular salts thereof, particular solvates thereof, and solvates of the particular salts. Such salts may be, for example, in the form of a pharmaceutically acceptable salt. Types of the sales are not particularly limited. However, salts may be in a safe and effective form in mammals, without being limited thereto.

The descriptions given above may also be applied to other specific embodiments or other aspects, without being limited thereto.

The pharmaceutical composition of the present invention may further include a pharmaceutically acceptable carrier, excipient, or diluent. The pharmaceutically acceptable carrier, excipient, or diluent may be one which is not naturally occurring.

As used herein, the term "pharmaceutically acceptable" refers to an amount sufficient for exhibiting therapeutic effects without causing side effects and may be easily determined by one or ordinary skill in the art based on factors well-known in the medical field such as the type of disease, age, body weight, health status, and gender of a patient, and patient's sensitivity to drug, administration route, administration method, frequency of administration, duration of treatment, and a drug used in combination or concurrently.

The pharmaceutical composition of the present invention may further include a pharmaceutically acceptable carrier. As the pharmaceutically acceptable carrier is a buffer, a preservative, an analgesic, a solubilizer, an isotonic agent, and a stabilizer may be used in combination, without being limited thereto.

The pharmaceutical composition of the present invention includes the triple agonist or the long-acting conjugate thereof in a pharmacologically effective amount, specifically at a dose of 0.5 mg to 8 mg, and is administered to a patient with obesity and/or a non-alcoholic fatty liver disease once a week.

Such administration once a week at a dose of 0.5 mg to 8 mg is technically significant in that these are optimal dosing interval and dose to obtain efficacy, drug tolerance, and stability proved from via clinical trials conducted on patients with obesity and/or a non-alcoholic fatty liver disease and substantive preventive and therapeutic effects on obesity and/or non-alcoholic fatty liver disease may be obtained by administering the pharmaceutical composition of the present invention.

Another aspect of the present invention provides a method of preventing or treating obesity and/or a non-alcoholic fatty liver disease, the method including administering the long-acting conjugate of the triple agonist or a pharmaceutical composition including the same to a patient with obesity and/or a non-alcoholic fatty liver disease.

Specifically, the method of preventing or treating the obesity and/or a non-alcoholic fatty liver disease includes administering the long-acting conjugate of the triple agonist to the patient with obesity and/or a non-alcoholic fatty liver disease once a week at a dose of 0.5 mg to 8 mg via parenteral administration (particularly, subcutaneous administration), without being limited thereto.

The triple agonist, long-acting conjugate, pharmaceutical composition, obesity, non-alcoholic fatty liver disease, and administration are as described above.

Another aspect of the present invention provides a use of the long-acting conjugate of the triple agonist or the composition including the same in preparation of medicaments for preventing or treating obesity and/or a non-alcoholic fatty liver disease.

Specifically, the long-acting conjugate of the triple agonist may be administered to a patient with obesity and/or a non-alcoholic fatty liver disease once a week at a dose of 0.5 mg to 8 mg via parenteral administration (particularly, subcutaneous administration), without being limited thereto.

The triple agonist, long-acting conjugate, pharmaceutical composition, obesity, non-alcoholic fatty liver disease, and administration are as described above.

Another aspect of the present invention provides a preparation for preventing or treating obesity and/or a non-alcoholic fatty liver disease including the long-acting conjugate of the triple agonist.

Specifically, the preparation is administered to a patient with obesity and/or a non-alcoholic fatty liver disease once a week at a dose of 0.5 mg to 8 mg via parenteral administration (particularly, subcutaneous administration), without being limited thereto.

The triple agonist, long-acting conjugate, pharmaceutical composition, obesity, non-alcoholic fatty liver disease, and administration are as described above.

Hereinafter, the present invention will be described in more detail with reference to the following examples. However, the following examples are merely presented to exemplify the present invention, and the scope of the present invention is not limited thereto.

### Example 1: Preparation of Triple Agonist

Triple agonists having activities to all of GLP-1, GIP, and glucagon receptors were prepared, and sequences thereof are listed in Table 1 below.

**Table 1**

| **SEQ ID NO:** | **Sequence** | **Information** |
|---|---|---|
| 1 | H X Q G T F T S D V S S Y L D G Q A A K E F I A W L V K G C | |
| 2 | H X Q G T F T S D V S S Y L D G Q A Q K E F I A W L V K G C | |
| 3 | H X Q G T F T S D V S S Y L L G Q A A K Q F I A W L V K G G G P S S G A P P P S C | |
| 4 | H X Q G T F T S D V S S Y L L G Q Q Q K E F I A W L V K G C | |
| 5 | H X Q G T F T S D V S S Y L L G Q Q Q K E F I A W L V K G G G P S S G A P P P S C | |
| 6 | H X Q G T F T S D V S S Y L D G Q A A K E F V A W L L K G C | |
| 7 | H X Q G T F T S D V S K Y L D G Q A A K E F V A W L L K G C | |
| 8 | H X Q G T F T S D V S K Y L D G Q A A Q E F V A W L L K G C | |
| 9 | H X Q G T F T S D V S K Y L D G Q A A Q E F V A W L L A G C | |
| 10 | HXQGTFTSDVSKYLDGQAAQEFVAWLLA GGGPSSGAPPPSC | |
| 11 | CAGEGTFTSDLSKYLDSRRQQLFVQWLKA GGPSSGAPPPSHG | |
| 12 | CAGEGTFISDLSKYMDEQAVQLFVEWLMA GGPSSGAPPPSHG | |
| 13 | CA G E G T F I S D Y S I Q L D E I A V Q D F V E W L L A Q K P S S G A P P P S H G | |
| 14 | CA G Q G T F T S D Y S I Q L D E I A V R D F V E W L K N G G P S S G A P P P S H G | |
| 15 | CAGQGTFTSDLSKQMDEEAVRLFIEWLKN GGPSSGAPPPSHG | |
| 16 | CA G Q G T F T S D L S K Q M D S E A Q Q L F I E W L K N G G P S S G A P P P S H G | |
| 17 | CAGQGTFTSDLSKQMDEERAREFIEWLLA QKPSSGAPPPSHG | |
| 18 | CA G Q G T F T S D L S K Q M D S E R A R E F I E W L K N T G P S S G A P P P S H G | |
| 19 | CA G Q G T F T S D L S I Q Y D S E H Q R D F I E W L K D T G P S S G A P P P S H G | |
| 20 | CA G Q G T F T S D L S I Q Y E E E A Q Q D F V E W L K D T G P S S G A P P P S H G | |
| 21 | Y X Q G T F T S D Y S K Y L D E C R A K E F V Q W L L D H H P S S G Q P P P S | ring formed |
| 22 | Y X Q G T F T S D Y S K C L D E K R A K E F V Q W L L D H H P S S G Q P P P S | ring formed |
| 23 | Y X Q G T F T S D Y S K Y L D E C R A K E F V Q W L L A Q K G K K N D W K H N I T | ring formed |
| 24 | Y X Q G T F T S D Y S K Y L D E C R A K E F V Q W L K N G G P S S G A P P P S | ring formed |
| 25 | H X Q G T F T S D C S K Y L D E R A A Q D F V Q W L L D G G P S S G A P P P S | |
| 26 | H X Q G T F T S D C S K Y L D S R A A Q D F V Q W L L D G G P S S G A P P P S | |
| 27 | H X Q G T F T S D Y S K Y L D E R A C Q D F V Q W L L D Q G G P S S G A P P P S | |
| 28 | H X Q G T F T S D Y S K Y L D E K R A Q E F V C W L L A Q K G K K N D W K H N I T | |
| 29 | H X Q G T F T S D Y S K Y L D E K A A K E F V Q W L L N T C | ring formed |
| 30 | HXQGTFTSDYSKYLDEKAQKEFVQWLLDT C | ring formed |
| 31 | H X Q G T F T S D Y S K Y L D E K A C K E F V Q W L L A Q | ring formed |
| 32 | H X Q G T F T S D Y S K Y L D E K A C K D F V Q W L L D G G P S S G A P P P S | ring formed |
| 33 | H X Q G T F T S D Y S I A M D E I H Q K D F V N W L L A Q | ring formed |
| | K C | |
| 34 | H X Q G T F T S D Y S K Y L D E K R Q K E F V N W L L A Q K C | ring formed |
| 35 | H X Q G T F T S D Y S I A M D E I H Q K D F V N W L L N T K C | ring formed |
| 36 | H X Q G T F T S D Y S K Y L C E K R Q K E F V Q W L L N G G P S S G A P P P S G | ring formed |
| 37 | HXQGTFTSDYSKYLDECRQKEFVQWLLN GGPSSGAPPPSG | ring formed |
| 38 | CAXQGTFTSDKSSYLDERAAQDFVQWLLD GGPSSGAPPPSS | |
| 39 | H X Q G T F T S D Y S K Y L D G Q H A Q C F V A W L L A G G G P S S G A P P P S | |
| 40 | H X Q G T F T S D K S K Y L D E R A C Q D F V Q W L L D G G P S S G A P P P S | |
| 41 | H X Q G T F T S D K S K Y L D E C A A Q D F V Q W L L D G G P S S G A P P P S | |
| 42 | Y X Q G T F T S D Y S K Y L D E K R A K E F V Q W L L D H H P S S G Q P P P S C | ring formed |
| 43 | Y X Q G T F T S D Y S K Y L D E K R A K E F V Q W L L D H H C S S G Q P P P S | ring formed |
| 44 | H G Q G T F T S D C S K Q L D G Q A A Q E F V A W L L A G G P S S G A P P P S | |
| 45 | H G Q G T F T S D C S K Y M D G Q A A Q D F V A W L L A G G P S S G A P P P S | |
| 46 | H G Q G T F T S D C S K Y L D E Q H A Q E F V A W L L A G G P S S G A P P P S | |
| 47 | H G Q G T F T S D C S K Y L D G Q R A Q E F V A W L L A G G P S S G A P P P S | |
| 48 | H G Q G T F T S D C S K Y L D G Q R A Q D F V N W L L A G G P S S G A P P P S | |
| 49 | CA X Q G T F T S D Y S I C M D E I H Q K D F V N W L L N | ring formed |
| | T K | |
| 50 | H X Q G T F T S D Y S K Y L D E K R A K E F V Q W L L D H H P S S G Q P P P S C | ring formed |
| 51 | H X Q G T F T S D Y S K Y L D E K R Q K E F V Q W L L N T C | ring formed |
| 52 | H X Q G T F T S D Y S K Y L D E K R Q K E F V Q W L L D T C | ring formed |
| 53 | H X E G T F T S D Y S I A M D E I H Q K D F V N W L L A Q C | ring formed |
| 54 | H X E G T F T S D Y S I A M D E I H Q K D F V D W L L A E C | ring formed |
| 55 | H X Q G T F T S D Y S I A M D E I H Q K D F V N W L L A Q C | ring formed |
| 56 | H X Q G T F T S D Y S K Y L D E K R Q K E F V N W L L A Q C | ring formed |
| 57 | H X Q G T F T S D Y S I A M D E I H Q K D F V N W L L N T C | ring formed |
| 58 | H X Q G T F T S D Y S K Y L D E K R Q K E F V Q W L L N T K C | ring formed |
| 59 | CAXQGTFTSDYSICMDEKHQKDFVNWLLN TK | ring formed |
| 60 | CA X Q G T F T S D Y S I A M D E K H C K D F V N W L L N T K | ring formed |
| 61 | CA X Q G T F T S D Y S I A M D E I A C K D F V N W L L N T K | ring formed |
| 62 | CAXQGTFTSDKSKYLDERAAQDFVQWLLD GGPSSGAPPPS | - |
| 63 | CAXQGTFTSDCSKYLDERAAQDFVQWLLD GGPSSGAPPPS | - |
| 64 | Y X Q G T F T S D Y S K Y L D E C A A K E F V Q W L L D H H P S S G Q P P P S | ring formed |
| 65 | H X Q G T F T S D Y S K C L D E K R A K E F V Q W L L D | ring formed |
| | H H P S S G Q P P P S | |
| 66 | Y X Q G T F T S D Y S K Y L D E C R A K D F V Q W L L D H H P S S G Q P P P S | ring formed |
| 67 | Y X Q G T F T S D Y S K Y L D E C A A K D F V Q W L L D H H P S S G Q P P P S | ring formed |
| 68 | Y X Q G T F T S D Y S K C L D E K A A K E F V Q W L L D H H P S S G Q P P P S | ring formed |
| 69 | Y X Q G T F T S D Y S K C L D E R A A K E F V Q W L L D H H P S S G Q P P P S | ring formed |
| 70 | Y X Q G T F T S D Y S K C L D E K R A K D F V Q W L L D H H P S S G Q P P P S | ring formed |
| 71 | Y X Q G T F T S D Y S K Y L D E R A C K D F V Q W L L D H H P S S G Q P P P S | ring formed |
| 72 | Y X Q G T F T S D C S K Y L D E R A A K D F V Q W L L D H H P S S G Q P P P S | ring formed |
| 73 | CA X Q G T F T S D Y S K Y L D E C R A K E F V Q W L L D H H P S S G Q P P P S | ring formed |
| 74 | CA X Q G T F T S D Y S K C L D E K R A K E F V Q W L L D H H P S S G Q P P P S | ring formed |
| 75 | Y X Q G T F T S D Y S K Y L D E K A A K E F V Q W L L D H H P S S G Q P P P S C | ring formed |
| 76 | Y X Q G T F T S D Y S K Y L D E K R A K D F V Q W L L D H H P S S G Q P P P S C | ring formed |
| 77 | Y X Q G T F T S D Y S K Y L D E K A A K D F V Q W L L D H H P S S G Q P P P S C | ring formed |
| 78 | H X Q G T F T S D Y S K Y L D E K R Q K E F V Q W L L D T K C | ring formed |
| 79 | H X E G T F T S D Y S I A M D E I H Q K D F V N W L L A Q K C | ring formed |
| 80 | H X E G T F T S D Y S I A M D E I H Q K D F V D W L L A E K C | ring formed |
| 81 | CAXQGTFTSDYSKYLDEKRQKEFVQWLLN | ring formed |
| | T C | |
| 82 | CA X Q G T F T S D Y S K Y L D E K R Q K E F V Q W L L D T C | ring formed |
| 83 | CA X E G T F T S D Y S I A M D E I H Q K D F V N W L L A Q C | ring formed |
| 84 | CA X E G T F T S D Y S I A M D E I H Q K D F V D W L L A E C | ring formed |
| 85 | CA X Q G T F T S D Y S I A M D E I H Q K D F V N W L L A Q C | ring formed |
| 86 | CA X Q G T F T S D Y S K Y L D E K R Q K E F V N W L L A Q C | ring formed |
| 87 | CA X Q G T F T S D Y S I A M D E I H Q K D F V N W L L N T C | ring formed |
| 88 | CAXQGTFTSDYSKYLDEKRQKEFVQWLLN TKC | ring formed |
| 89 | CAXQGTFTSDYSKYLDEKRQKEFVQWLLD TKC | ring formed |
| 90 | CA X E G T F T S D Y S I A M D E I H Q K D F V N W L L A Q K C | ring formed |
| 91 | CA X E G T F T S D Y S I A M D E I H Q K D F V D W L L A E K C | ring formed |
| 92 | CA X Q G T F T S D Y S I A M D E I H Q K D F V N W L L A Q K C | ring formed |
| 93 | CA X Q G T F T S D Y S K Y L D E K R Q K E F V N W L L A Q K C | ring formed |
| 94 | CA X Q G T F T S D Y S I A M D E I H Q K D F V N W L L N T K C | ring formed |
| 95 | Y X Q G T F T S D Y S K Y L D E K R A K E F V Q W L L C H H P S S G Q P P P S | ring formed |
| 96 | Y X Q G T F T S D Y S K Y L D E K R A K E F V Q W L L D H C P S S G Q P P P S | ring formed |
| 97 | Y X Q G T F T S D Y S K Y L D E K R A K E F V Q W L L D C | ring formed |
| | H P S S G Q P P P S | |
| 98 | Y X Q G T F T S D Y S K A L D E K A A K E F V N W L L D H H P S S G Q P P P S C | ring formed |
| 99 | Y X Q G T F T S D Y S K A L D E K A A K D F V N W L L D H H P S S G Q P P P S C | ring formed |
| 100 | Y X Q G T F T S D Y S K A L D E K A A K E F V Q W L L D Q H P S S G Q P P P S C | ring formed |
| 101 | Y X Q G T F T S D Y S K A L D E K A A K E F V N W L L D Q H P S S G Q P P P S C | ring formed |
| 102 | Y X Q G T F T S D Y S K A L D E K A A K D F V N W L L D Q H P S S G Q P P P S C | ring formed |

In the sequences shown in Table 1, the amino acid marked with X represents aminoisobutyric acid (Aib), which is a non-native amino acid, and the underlined amino acids represent formation of a ring therebetween. Also, in Table 1, CA is 4-imidazoacetyl, and Y is tyrosine.

### Example 2: Preparation of Long-acting Conjugate of Triple Agonist

For pegylation of 10 kDa PEG having a maleimide group and an aldehyde group at both ends respectively, *i.e*., maleimide-PEG-aldehyde (10 kDa, NOF, Japan), into a cysteine residue of each of the triple agonists of Example 1 (SEQ ID NOS: 21, 22, 42, 43, 50, 77, and 96), the triple agonist and the maleimide-PEG-aldehyde were reacted at a molar ratio of 1:1 to 3 with a protein concentration of 1 mg/mL to 5 mg/mL at a low temperature for 0.5 to 3 hours. In this case, the reaction was conducted in an environment including 50 mM Tris buffer (pH 7.5) to which 20% to 60% isopropanol was added. Upon termination of the reaction, the reaction solution was applied to SP Sepharose HP (GE Healthcare, USA) to purify the triple agonist mono-pegylated on cysteine.

Subsequently, the purified mono-pegylated triple agonist and an immunoglobulin Fc (homodimer of SEQ ID NO: 123) were reacted at a molar ratio of 1:1 to 5 with a protein concentration of 10 mg/mL to 50 mg/mL at a temperature of 4°C to 8°C for 12 to 18 hours. The reaction was conducted in an environment in which a 10 mM to 50 mM sodium cyanoborohydride, as a reducing agent, and a 10% to 30% isopropanol were added to a 100 mM calcium phosphate buffer (pH 6.0). Upon termination of the reaction, the reactant solution was applied to a Butyl Sepharose FF Purification Column (GE Healthcare, USA) and a Source ISO purification column (GE Healthcare, USA) to purify the conjugate including the triple agonist and the immunoglobulin Fc.

Meanwhile, the immunoglobulin Fc may be in the form of a homodimer in which two monomers each having an amino acid sequence of SEQ ID NO: 123 (consisting of 221 amino acids) are linked to each other via a disulfide bond between cysteines at position 3 of each monomer, wherein the monomers of the homodimer each independently have a disulfide bond formed between cysteines at positions 35 and 95 and a disulfide bond formed between cysteines at positions 141 and 199, without being limited thereto.

After the preparation, a purity analyzed by reverse phase chromatography, size exclusion chromatography, and ion exchange chromatography was 95% or more.

In this regard, a conjugate in which the triple agonist of SEQ ID NO: 21 is linked to the immunoglobulin Fc via the PEG linker was named "conjugate including SEQ ID NO: 21 and immunoglobulin Fc" or "long-acting conjugate of SEQ ID NO: 21", and they may be used interchangeably in the present invention.

In this regard, a conjugate in which the triple agonist of SEQ ID NO: 22 is linked to the immunoglobulin Fc via the PEG linker was named "conjugate including SEQ ID NO: 22 and immunoglobulin Fc" or "long-acting conjugate of SEQ ID NO: 22", and they may be used interchangeably in the present invention.

In this regard, a conjugate in which the triple agonist of SEQ ID NO: 42 is linked to the immunoglobulin Fc via the PEG linker was named "conjugate including SEQ ID NO: 42 and immunoglobulin Fc" or "long-acting conjugate of SEQ ID NO: 42", and they may be used interchangeably in the present invention.

In this regard, a conjugate in which the triple agonist of SEQ ID NO: 43 is linked to the immunoglobulin Fc via the PEG linker was named "conjugate including SEQ ID NO: 43 and immunoglobulin Fc" or "long-acting conjugate of SEQ ID NO: 43", and they may be used interchangeably in the present invention.

In this regard, a conjugate in which the triple agonist of SEQ ID NO: 50 is linked to the immunoglobulin Fc via the PEG linker was named "conjugate including SEQ ID NO: 50 and immunoglobulin Fc" or "long-acting conjugate of SEQ ID NO: 50", and they may be used interchangeably in the present invention.

In this regard, a conjugate in which the triple agonist of SEQ ID NO: 77 is linked to the immunoglobulin Fc via the PEG linker was named "conjugate including SEQ ID NO: 77 and immunoglobulin Fc" or "long-acting conjugate of SEQ ID NO: 77", and they may be used interchangeably in the present invention.

In this regard, a conjugate in which the triple agonist of SEQ ID NO: 96 is linked to the immunoglobulin Fc via the PEG linker was named "conjugate including SEQ ID NO: 96 and immunoglobulin Fc" or "long-acting conjugate of SEQ ID NO: 96", and they may be used interchangeably in the present invention.

### Experimental Example 1: Measurement of In Vitro Activity of Triple Agonist and Long-acting Conjugate Thereof

Activities of the triple agonists and the long-acting conjugates thereof respectively prepared in Examples 1 and 2 were measured using transformed cell lines in which a GLP-1 receptor, a glucagon (GCG) receptor, and a GIP receptor were transformed by way of a method of measuring *in vitro* cellular activities.

The cell lines were transformed such that genes for the human GLP-1 receptor, the human GCG receptor, and the human GIP receptor were each expressed in Chinese hamster ovary (CHO), and were suitable for measuring activities of GLP-1, GCG, and GIP. Therefore, the activity for each part was measured using each of the transformed cell lines.

For measurement of the activity of each of the triple agonists and the long-acting conjugates thereof respectively prepared in Examples 1 and 2 to GLP-1, human GLP-1 was subjected to a 4-fold serial dilution from 50 nM to 0.000048 nM, and the triple agonists and the long-acting conjugates thereof respectively prepared in Examples 1 and 2 were subjected to a 4-fold serial dilution from 400 nM to 0.00038 nM. A culture broth was removed from the cultured CHO cells in which the human GLP-1 receptor was expressed, and each of the serially diluted substances was added to the CHO cells in an amount of 5 µL. Thereafter, a buffer solution including cAMP antibody was added thereto in an amount of 5 µL and cultured at room temperature for 15 minutes. Then, a detection mix including a cell lysis buffer was added thereto in an amount of 10 µL for lysis of the cells, followed by reaction at room temperature for 90 minutes. Upon termination of the reaction, the cell lysates were applied to a LANCE cAMP kit (PerkinElmer, USA) to calculate EC₅₀ values via accumulated cAMP, and the values were compared with each other. Relative potencies compared to human GLP-1 are shown in Tables 2 and 3 below.

For measurement of the activity of the triple agonists and the long-acting conjugates thereof respectively prepared in Examples 1 and 2 to GCG, human GCG was subjected to a 4-fold serial dilution from 50 nM to 0.000048 nM, and the triple agonists and the long-acting conjugates thereof respectively prepared in Examples 1 and 2 were subjected to a 4-fold serial dilution from 400 nM to 0.00038 nM. A cultured broth was removed from the cultured CHO cells in which the human GCG receptor was expressed, and each of the serially diluted substances was added to the CHO cells in an amount of 5 µL. Then, a buffer solution including cAMP antibody was added thereto in an amount of 5 µL and cultured at room temperature for 15 minutes. Then, a detection mix including a cell lysis buffer was added thereto in an amount of 10 µL for lysis of the cells, followed by reaction at room temperature for 90 minutes. Upon termination of the reaction, the cell lysates were applied to a LANCE cAMP kit (PerkinElmer, USA) to calculate EC₅₀ values via accumulated cAMP, and the values were compared with each other. Relative potencies compared to human GCG are shown in Tables 2 and 3 below.

For measurement of the activity of the triple agonists and the long-acting conjugates thereof respectively prepared in Examples 1 and 2 to GIP, human GIP was subjected to a 4-fold serial dilution from 50 nM to 0.000048 nM, and the triple agonists and the long-acting conjugates thereof respectively prepared in Examples 1 and 2 were subjected to a 4-fold serial dilution from 400 nM to 0.00038 nM. A cultured broth was removed from the cultured CHO cells in which the human GIP receptor was expressed, and each of the serially diluted substances was added to the CHO cells in an amount of 5 µL. Then, a buffer solution including cAMP antibody was added thereto in an amount of 5 µL and cultured at room temperature for 15 minutes. Then, a detection mix including a cell lysis buffer was added thereto in an amount of 10 µL for lysis of the cells, followed by reaction at room temperature for 90 minutes. Upon termination of the reaction, the cell lysates were applied to a LANCE cAMP kit (PerkinElmer, USA) to calculate EC₅₀ values via accumulated cAMP, and the values were compared with each other. Relative potencies compared to human GIP are shown in Tables 2 and 3 below.

**Table 2**

| Relative potency ratio of triple agonist | | | |
|---|---|---|---|
| | *In vitro* activity relative to native peptide (%) | | |
| **SEQ ID NO:** | *vs*. GLP-1 | *vs*. Glucagon | *vs*. GIP |
| 1 | 3.2 | <0.1 | <0.1 |
| 2 | 5.9 | <0.1 | <0.1 |
| 3 | 1.8 | <0.1 | <0.1 |
| 4 | 8.5 | <0.1 | <0.1 |
| 5 | 42.1 | <0.1 | <0.1 |
| 6 | 17.0 | <0.1 | <0.1 |
| 7 | 13.7 | <0.1 | <0.1 |
| 8 | 14.2 | 0.10 | <0.1 |
| 9 | 32.1 | 0.13 | <0.1 |
| 10 | 46.0 | <0.1 | <0.1 |
| 11 | 1.4 | <0.1 | <0.1 |
| 12 | 0.4 | <0.1 | <0.1 |
| 13 | <0.1 | <0.1 | <0.1 |
| 14 | 28.0 | <0.1 | <0.1 |
| 15 | 79.2 | <0.1 | <0.1 |
| 16 | 2.1 | <0.1 | <0.1 |
| 17 | 0.2 | <0.1 | <0.1 |
| 18 | <0.1 | <0.1 | <0.1 |
| 19 | <0.1 | <0.1 | <0.1 |
| 20 | <0.1 | <0.1 | <0.1 |
| 21 | 17.8 | 267 | 22.7 |
| 22 | 20.1 | 140 | 59.7 |
| 23 | 4.01 | 9.3 | <0.1 |
| 24 | 41.2 | 9.3 | <0.1 |
| 25 | 82.6 | 0.1 | <0.1 |
| 26 | 64.5 | 0.2 | <0.1 |
| 27 | 83.1 | 0.8 | 0.9 |
| 28 | 17.2 | 1.6 | <0.1 |
| 29 | 38.5 | 6.0 | <0.1 |
| 30 | 142 | 0.7 | 0.8 |
| 31 | 135 | 2.2 | 2.4 |
| 32 | 151 | 1.7 | 8.8 |
| 33 | 24.5 | <0.1 | 10.4 |
| 34 | 19.1 | 0.92 | 0.6 |
| 35 | 7.5 | <0.1 | 1.3 |
| 36 | 37.4 | 0.39 | 0.2 |
| 37 | 236 | 6.21 | 2.2 |
| 38 | 2.3 | - | - |
| 39 | 13.9 | 0.53 | <0.1 |
| 40 | 75.2 | <0.1 | <0.1 |
| 41 | 34.3 | <0.1 | <0.1 |
| 42 | 33.9 | 205.8 | 7.8 |
| 43 | 12.6 | 88.4 | 3.70 |
| 44 | 1.3 | <0.1 | <0.1 |
| 45 | 6.6 | <0.1 | <0.1 |
| 46 | 1.4 | <0.1 | <0.1 |
| 47 | 2.4 | <0.1 | <0.1 |
| 48 | 1.5 | <0.1 | <0.1 |
| 49 | 29.8 | <0.1 | 3.3 |
| 50 | 67.4 | 50.5 | 2.7 |
| 51 | 14.4 | 2.0 | 0.1 |
| 52 | 44.1 | 7.5 | 0.3 |
| 53 | 161 | 8.4 | 1.3 |
| 54 | 30.6 | 1.4 | 0.1 |
| 55 | 27.1 | 0.7 | 2.4 |
| 56 | 57.9 | 4.9 | 0.8 |
| 57 | 11.7 | <0.1 | 0.3 |
| 58 | 39.1 | 2.6 | 0.2 |
| 59 | 40.3 | <0.1 | 4.0 |
| 60 | 106.2 | <0.1 | 8.2 |
| 61 | 59.8 | <0.1 | 2.8 |
| 62 | 5.2 | <0.1 | <0.1 |
| 63 | 15.3 | <0.1 | <0.1 |
| 64 | 64.6 | 60.1 | 92.9 |
| 65 | 95.4 | 25.2 | 11.6 |
| 66 | 15.8 | 172 | 17.2 |
| 67 | 28.5 | 46.2 | 39.8 |
| 68 | 27.9 | 8.8 | 107 |
| 69 | 24.3 | 9.6 | 62.8 |
| 70 | 15.1 | 71.3 | 64.4 |
| 71 | 90.1 | 12.7 | 94.7 |
| 72 | 11.5 | 1.0 | 1.6 |
| 73 | 22.6 | 5.4 | 3.0 |
| 74 | 12.9 | 0.9 | 1.0 |
| 75 | 35.1 | 8.5 | 18.0 |
| 76 | 10.3 | 47.6 | 11.7 |
| 77 | 38.7 | 12.2 | 35.5 |
| 78 | 51.0 | 14.0 | 0.12 |
| 79 | 41.5 | 4.9 | 1.4 |
| 80 | 8.1 | 0.0 | 0.1 |
| 81 | 7.8 | 0.3 | <0.1 |
| 82 | 9.5 | 1.1 | <0.1 |
| 83 | 47.3 | 1.3 | 0.4 |
| 84 | 4.2 | <0.1 | <0.1 |
| 85 | 4.3 | <0.1 | 0.3 |
| 86 | 28.4 | 0.4 | 0.2 |
| 87 | 0.9 | <0.1 | <0.1 |
| 88 | 9.6 | 0.3 | <0.1 |
| 89 | 7.1 | 0.7 | <0.1 |
| 90 | 7.4 | <0.1 | <0.1 |
| 91 | 31.9 | 16.8 | 0.3 |
| 92 | 0.8 | <0.1 | 0.4 |
| 93 | 5.7 | 0.3 | 0.7 |
| 94 | 0.5 | <0.1 | <0.1 |
| 95 | 2.1 | 0.4 | <0.1 |
| 96 | 34.4 | 194.8 | 5.2 |
| 97 | 10.5 | 62.8 | 2.6 |
| 98 | 28.1 | 8.2 | 47.1 |
| 99 | 20.9 | 14.9 | 57.7 |
| 100 | 42.2 | 12.7 | 118.5 |
| 101 | 23.2 | 13.9 | 40.1 |
| 102 | 23.3 | 29.5 | 58.0 |

**Table 3**

| Relative potency ratio of long-acting conjugate of triple agonist | | | |
|---|---|---|---|
| Long-acting conjugate | *In vitro* activity relative to native peptide (%) | | |
| | *vs*. GLP-1 | *vs*. Glucagon | *vs*. GIP |
| 21 | 0.1 | 1.6 | 0.2 |
| 22 | 0.1 | 0.9 | 0.5 |
| 42 | 3.1 | 23.1 | 1.2 |
| 43 | 2.1 | 13.5 | 0.6 |
| 50 | 15.4 | 6.9 | 0.7 |
| 77 | 6.7 | 1.7 | 6.6 |
| 96 | 0.3 | 4.0 | 0.3 |

The novel long-acting conjugates of the triple agonists prepared as described above have a function as triple agonists capable of activating all of the GLP-1 receptor, the GIP receptor, and the glucagon receptor and may be used as a therapeutic agent for a patient with obesity and/or a non-alcoholic fatty liver disease.

### Experimental Example 2: Measurement of In Vitro Activity of Long-acting Conjugate of Triple Agonist

High-fat diet-induced obese mice, which are widely used as an obesity animal model, were used in this study. The body weights of the mice were in the range of about 50 g to 60 g before administration. The mice divided into groups were maintained in a casing system and had free access to water during this study. Lights were switched off from 6:00 P.M. to 6:00 A.M.

Experimental groups fed with a high-fat diet include: Group 1: administered with an excipient (via injection once every 2 days) - control of high-fat diet-induced obese mice, Group 2: administered with the long-acting conjugate of SEQ ID NO: 42, 1.44 nmol/kg (via injection once every 2 days), Group 3: administered with the long-acting conjugate of SEQ ID NO: 42, 2.88 nmol/kg (via injection once every 2 days), Group 4: administered with the long-acting conjugate of SEQ ID NO: 43, 1.44 nmol/kg (via injection once every 2 days), Group 5: administered with the long-acting conjugate of SEQ ID NO: 43, 2.88 nmol/kg (via injection once every 2 days), Group 6: administered with the long-acting conjugate of SEQ ID NO: 50, 1.44 nmol/kg (via injection once every 2 days), and Group 7: administered with the long-acting conjugate of SEQ ID NO: 50, 2.88 nmol/kg (via injection once every 2 days). The experiment was terminated on Day 28, and changes in body weight of the mice in each group were measured at 2-day intervals during the progress of the experiment. Upon termination of the experiment, the weight of mesenteric fat was measured by way of autopsy. Statistical analysis was performed by comparing the control of the high-fat diet-induced obese mice with the experimental groups by one-way AN OVA.

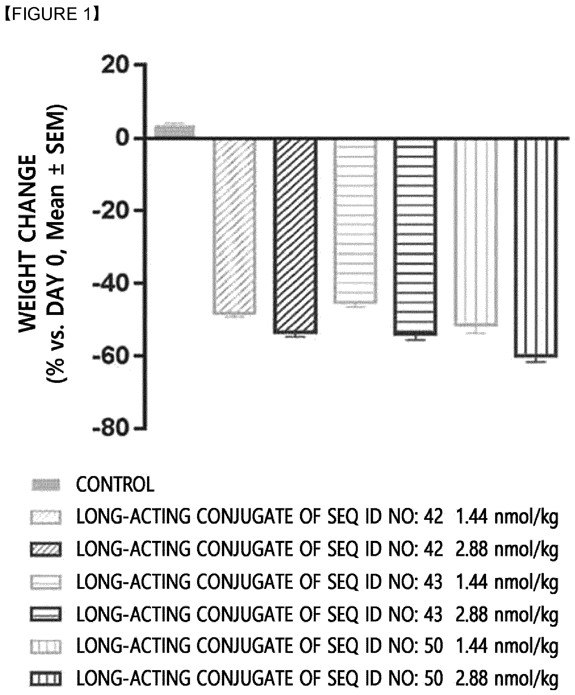

As a result of the measurement of changes in body weight, as shown in FIG. 1, it was confirmed that all of the groups administered with the high dose of the long-acting conjugates of SEQ ID NOS: 42, 43, and 50 exhibited weight loss by -56.9%, -57.0%, and -63.5%, respectively, on Day 28 after administration compared to the body weights before administration (***p <0.001 *vs*. excipient-administered control by one-way ANOVA).

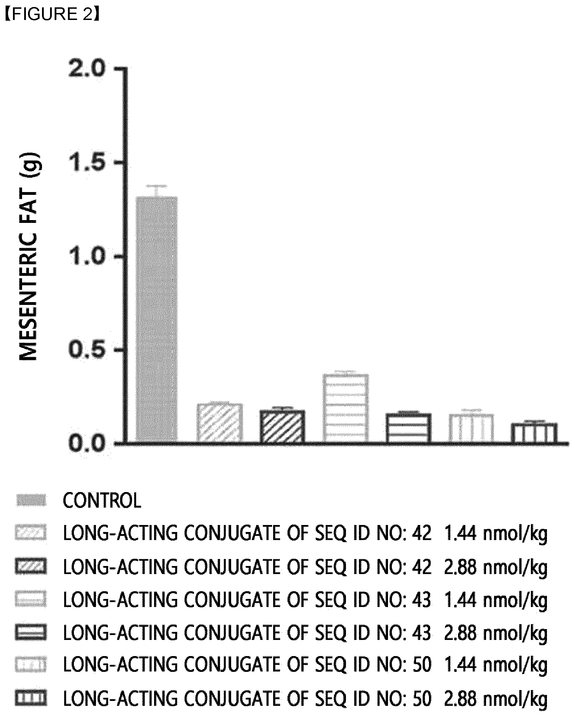

Also, as a result of measuring the weight of mesenteric fat, as shown in FIG. 2, it was confirmed that all of the groups administered with the high dose of the long-acting conjugates of SEQ ID NOS: 42, 43, and 50 exhibited significant decreases in fat in the body on Day 28 after administration compared to the group administered with the excipient (***p <0.001 vs. excipient-administered control by one-way ANOVA).

### Experimental Example 3: Experiment on Stability and Drug Tolerance of Long-acting Conjugate of Triple Agonist in Patient with Obesity

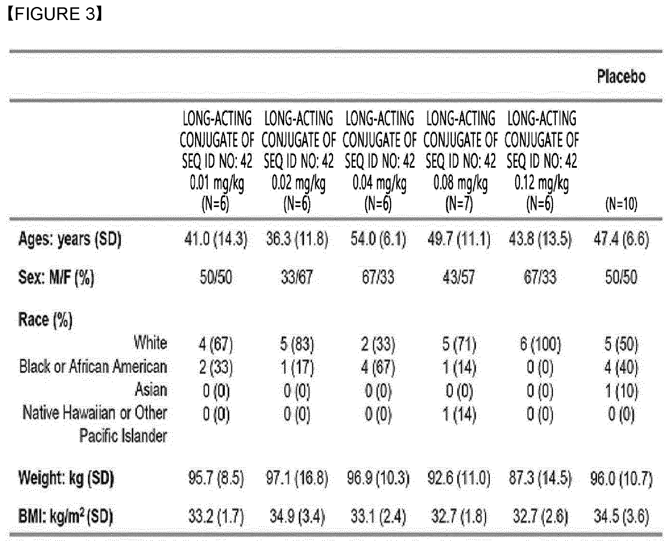

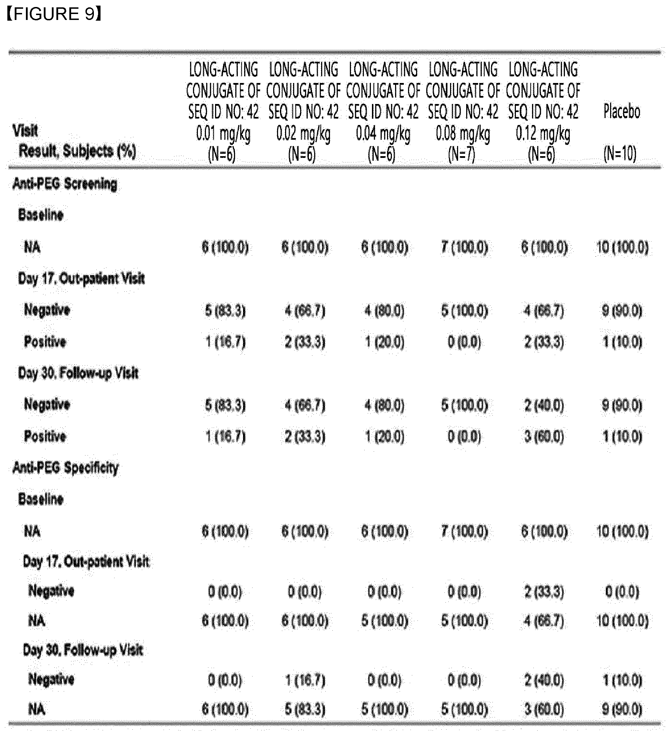

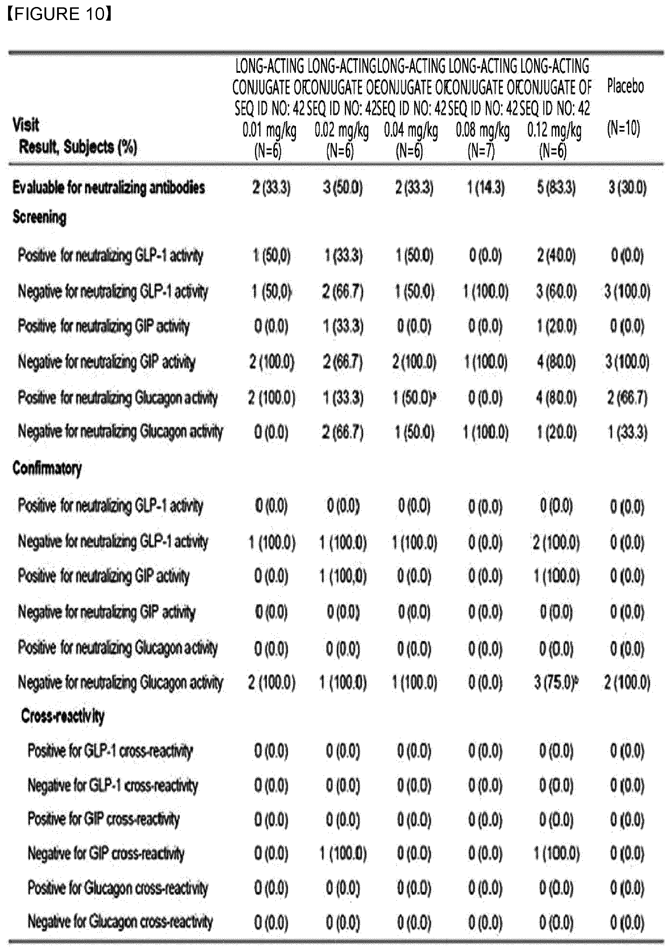

The long-acting conjugate of SEQ ID NO: 42 was subcutaneously administered to patients with obesity once at a dose of 0.01 mg/kg to 0.12 mg/kg. For one month after the administration, stability and drug tolerance were observed, and the results are shown in FIGS. 3 to 10.

Obese patients aged over 18 but less than 65 and having a BMI of 30 kg/m² to 40 kg/m² and an HbA1c less than 6.5% were collected. A total of 41 obese patients were collected. The average age of the 41 obese patients was 45.7, the average BMI was 33.6 kg/m², the ratio of males was 51.2%, and other information on the obese patients is as shown in FIG. 3. As shown in FIG. 3, except for seven obese patients administered at a dose of 0.08 mg/kg, each of the groups respectively administered at doses of 0.01 mg/kg, 0.02 mg/kg, 0.04 mg/kg, and 0.12 mg/kg included six obese patients. Obese patients of a placebo group were administered only with a sterile, colorless solution including a conjugate in which the immunoglobulin Fc is linked to PEG (not including the triple agonist of SEQ ID NO: 42), and a total of ten obese patients were administered such that two obese patients per group were administered with doses of 0.01 mg/kg, 0.02 mg/kg, 0.04 mg/kg, 0.08 mg/kg, and 0.12 mg/kg, respectively.

The condition of the obese patients was observed in a hospital from 2 days before administration to Day 7 after administration and observed as out-patients on Day 10 and Day 17, and then follow-up observations were conducted on Day 30.

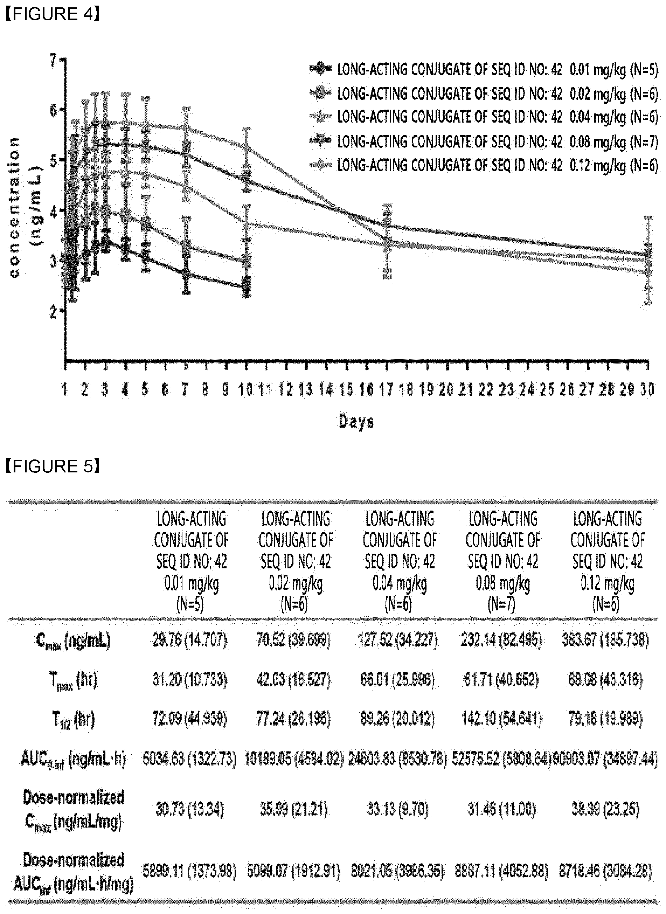

According to clinical trial manuals, 2 mL blood samples were collected from the obese patients on Day 1 (4 hours, 8 hours, and 12 hours after administration), on Day 2 (after 24 hours and 36 hours), on Day 3 (after 48 hours), on Day 4 (after 72 hours), on Day 5 (after 96 hours), on Day 7 (after 144 hours), on Day 10 (after 216 hours), on Day 17 (after 384 hours), and on Day 30 (after 696 hours). Blood concentration of the long-acting conjugate of SEQ ID NO: 42 was measured therefrom, and the results are shown in FIG. 4.

FIG. 5 shows Cₘₐₓ (ng/mL), Tₘₐₓ (hr), T_{1/2} (hr), AUC_{0-inf} (ng/mL·h), Dose-normalized Cₘₐₓ (ng/mL/mg), and Dose-normalized AUCinf (ng/mL·h/mg) when the long-acting conjugate of SEQ ID NO: 42 was administered.

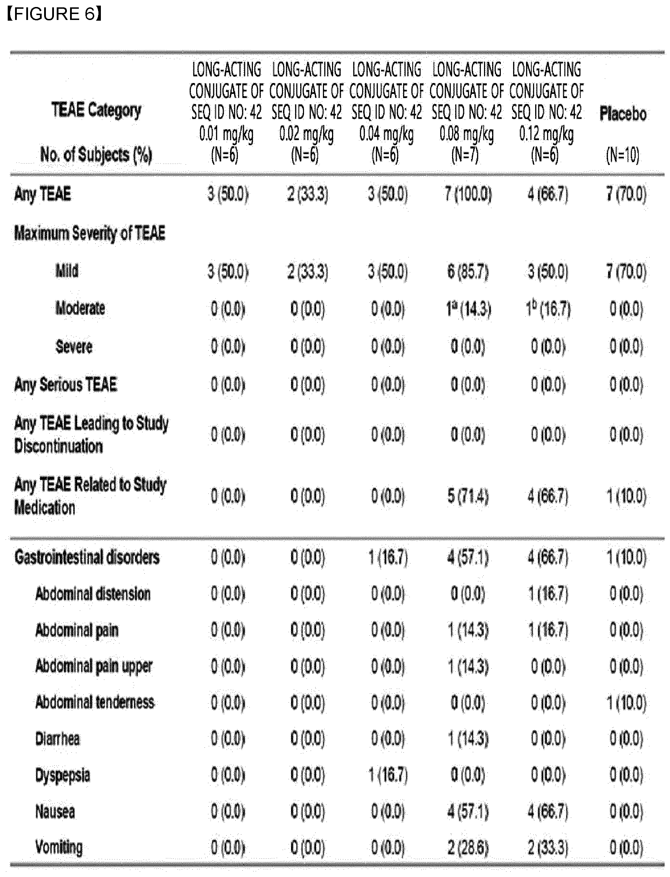

Treatment emergent adverse events (TEAEs) were observed for one month after administration, and the results are shown in FIG. 6.

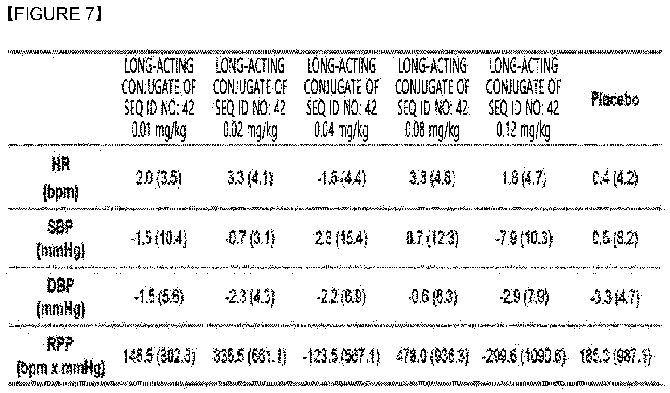

Blood pressure was continuously measured for 4 days by 24-hour ambulatory blood pressure monitoring (ABPM), and heart rate (HR), systolic blood pressure (SPB), diastolic blood pressure (DBP), and rate pressure product (RPP) are shown in FIG. 7.

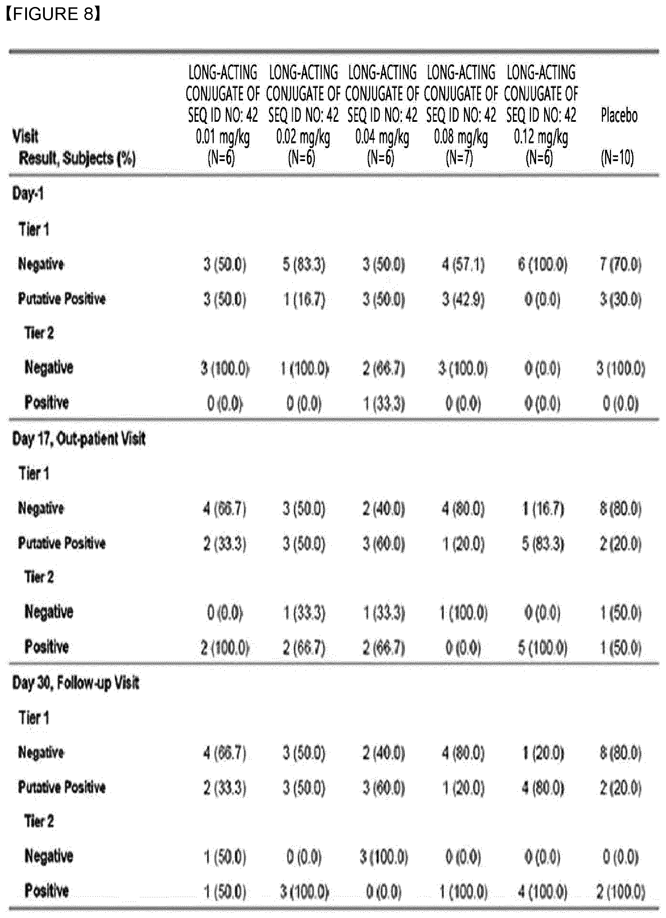

According to clinical trial manuals, 12 mL of blood samples were collected from the obese patients before administration (one day before administration) and on Day 7 (after 144 hours) and Day 30 (after 696 hours). Immunogenicity was measured (anti-drug antibodies (ADAbs); neutralizing antibodies (nAbs); and anti-polyethylene glycol antibodies (anti-PEG)), and the results are shown in FIGS. 8 to 10.

Based on the results, it was confirmed that stability and drug tolerance were secured when the long-acting conjugate was parenterally (subcutaneously) administered to the obese patients once a week at a dose of 0.5 mg to 8 mg.

### Experimental Example 4: Experiment on Stability, Drug Tolerance, and Efficacy of Long-acting Conjugate of Triple Agonist in Patient with Non-alcoholic Fatty Liver Disease

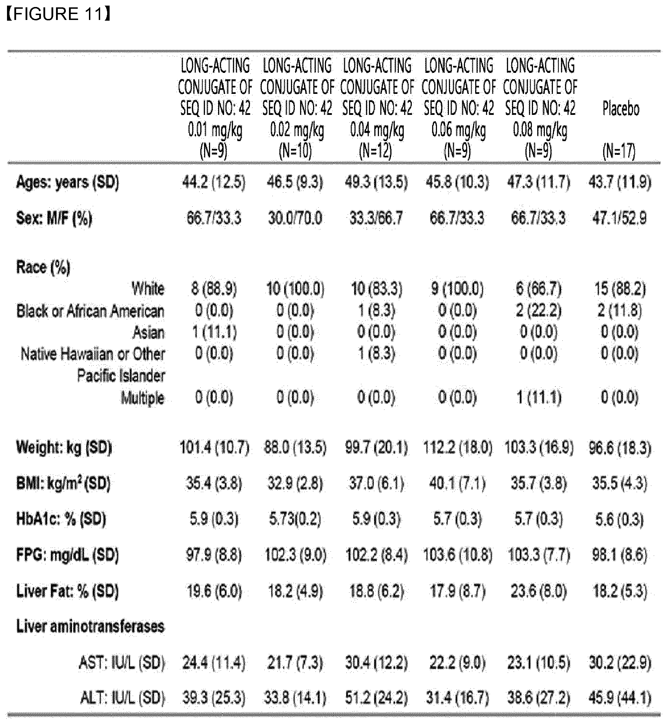

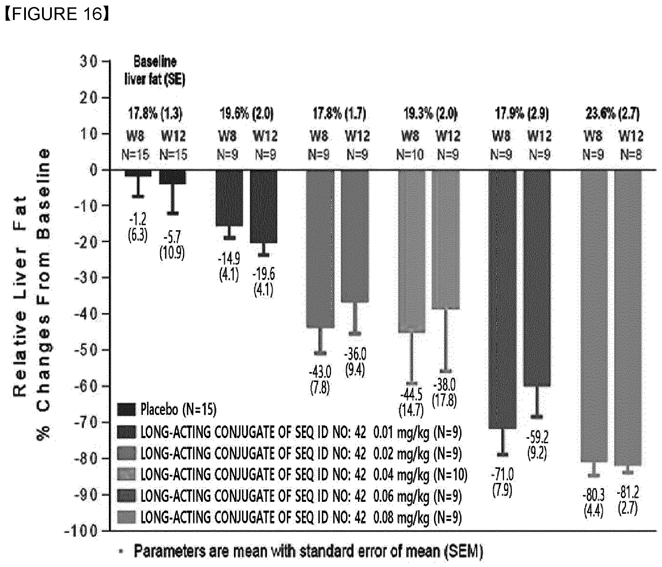

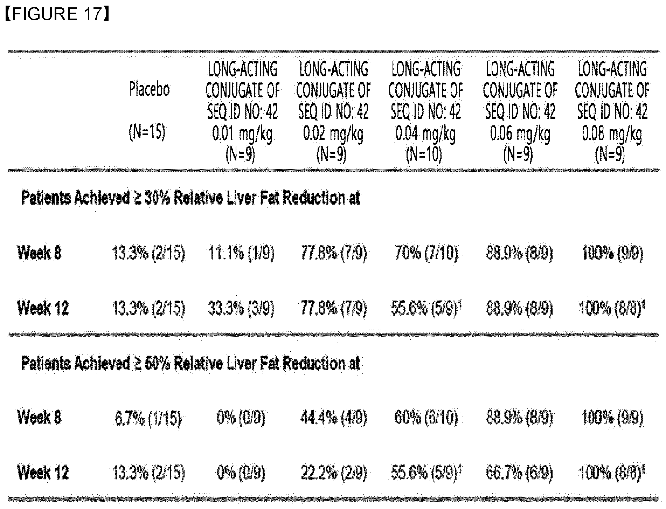

The long-acting conjugate of SEQ ID NO: 42 was subcutaneously administered to patients with a non-alcoholic fatty liver disease once a week at a dose of 0.01 mg/kg to 0.08 mg/kg for 12 weeks, and the results are shown in FIGS. 11 to 17.

Specifically, patients with a non-alcoholic fatty liver disease (NAFLD) having a BMI of 30 kg/m² or more, a waist size of 57 inches or less, a fasting plasma glucose level of 7 mmol/L (126 mg/dL) or less, an HbA1c less than 6.5%, a controlled attenuation parameter (CAP) value of 300 dB/m or more, which was measured by a fatty liver measuring technique using a FibroScan device, and a liver fat content of 10% or more, which was measured by MRI-PDFF, were collected. A total of 66 patients with a non-alcoholic fatty liver disease were collected. The ratio of females of the 66 patients with a non-alcoholic fatty liver disease was 50%, the average age was 46 (SD: 11.4), the average BMI was 36 kg/m² (SD: 4.96), the average liver fat content by MRI-PDFF was 19.2% (SD: 6.5), and other information on the patients with a non-alcoholic fatty liver disease is as shown in FIG. 11.

As shown in FIG. 11, the long-acting conjugate of SEQ ID NO: 42 was subcutaneously administered to the patients with a non-alcoholic fatty liver disease once a week for 12 weeks, at a dose of 0.01 mg/kg to nine patients, at a dose of 0.02 mg/kg to ten patients, at a dose of 0.04 mg/kg to twelve patients, at a dose of 0.06 mg/kg to nine patients, and at a dose of 0.08 mg/kg to nine patients. Patients with a non-alcoholic fatty liver disease of a placebo group were administered only with a sterile, colorless solution including a conjugate in which the immunoglobulin Fc is linked to PEG (not including the triple agonist of SEQ ID NO: 42), and a total of fifteen patients with a non-alcoholic fatty liver disease (three non-alcoholic fatty liver disease patients per each group) were administered at doses of 0.01 mg/kg, 0.02 mg/kg, 0.04 mg/kg, 0.06 mg/kg, and 0.08 mg/kg, respectively.

The condition of the patients with a non-alcoholic fatty liver disease was observed in a hospital from 2 days before administration to the 2^{nd} administration (Day 8 from initial administration) and observed as out-patients from at the 3^{rd} administration (Day 15 from initial administration), 4^{th} administration (Day 22 from initial administration), 5^{th} administration (Day 29 from initial administration), 6^{th} administration (Day 36 from initial administration), 7^{th} administration (Day 46 from initial administration), 8^{th} administration (Day 50 from initial administration), 9^{th} administration (Day 57 from initial administration), 10^{th} administration (Day 64 from initial administration), and 11^{th} administration (Day 71 from initial administration). The condition of the patients was observed in the hospital at the 12^{th} administration (Day 78 from initial administration). The condition was observed again as out-patients from Week 13 and Week 15 from initial administration and then followed up for 2 weeks thereafter.

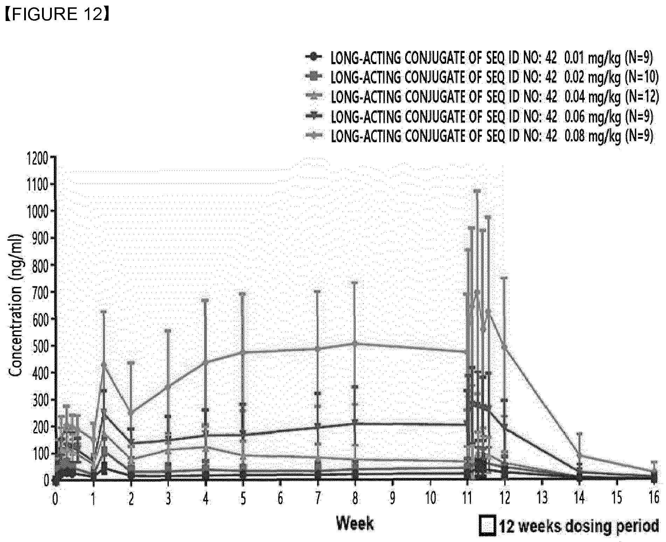

According to clinical trial manuals, 2 mL of blood samples were collected from the patients with a non-alcoholic fatty liver disease before administration and on Week 1 (8 hours, 24 hours, 48 hours, and 72 hours later from administration), on Week 2 (before the 2^{nd} administration and 48 hours after the 2^{nd} administration), on Week 3 (before the 3^{rd} administration and on Day 15 after initial administration) on Week 4 (before the 4^{th} administration and on Day 22 after initial administration), on Week 5 (before the 5^{th} administration and on Day 29 after initial administration), on Week 6 (before the 6^{th} administration and on Day 36 after initial administration), on Week 8 (before the 8^{th} administration and on Day 50 after initial administration), on Week 9 (before the 9^{th} administration and on Day 57 after initial administration), on Week 12 (before the 12^{th} administration, and 48 hours and 72 hours later from the 12^{th} administration), at 168 hours from the 12^{th} administration (on Day 85 after initial administration), at 504 hours from the 12^{th} administration (on Day 99 after initial administration), and at 840 hours from the 12^{th} administration (on Day 113 after initial administration). Blood concentration of the long-acting conjugate of SEQ ID NO: 42 were measured therefrom, and the results are shown in FIG. 12.

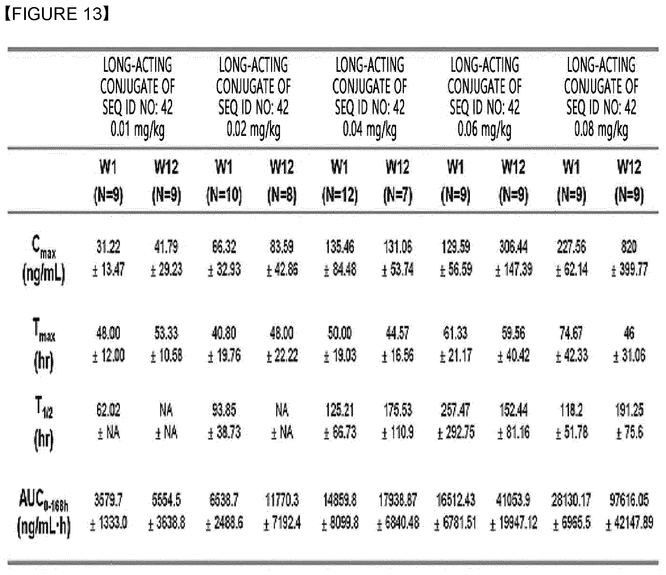

FIG. 13 shows Cₘₐₓ (ng/mL), Tₘₐₓ (hr), T_{1/2} (hr), and AUC_{0-inf} (ng/mL·h) confirmed on Week 1 and Week 12 after initial administration when the long-acting conjugate of SEQ ID NO: 42 is administered.

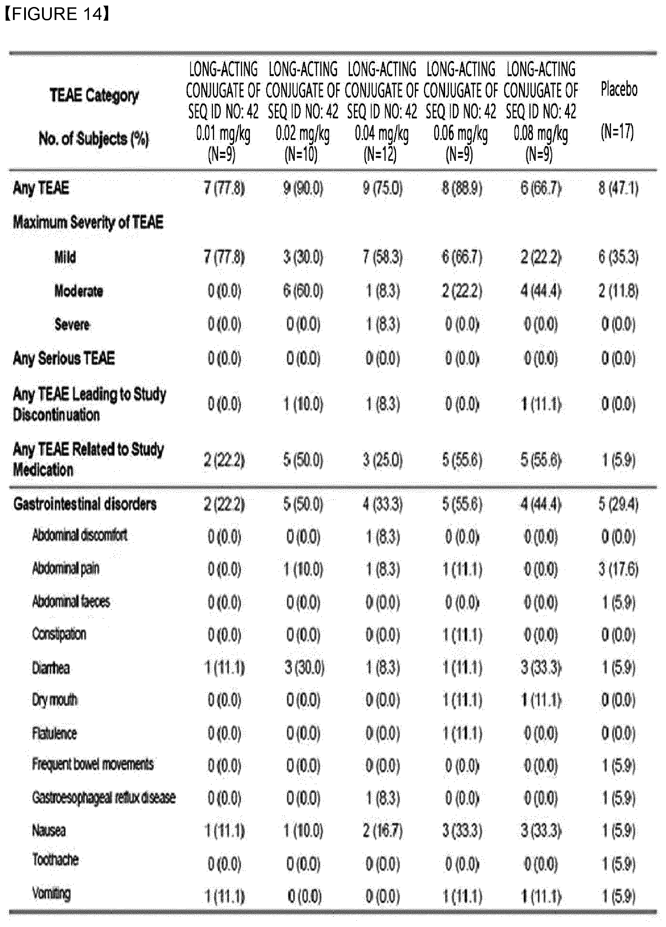

Treatment emergent adverse events (TEAEs) were observed until Week 17 after administration, and the results are shown in FIG. 14.

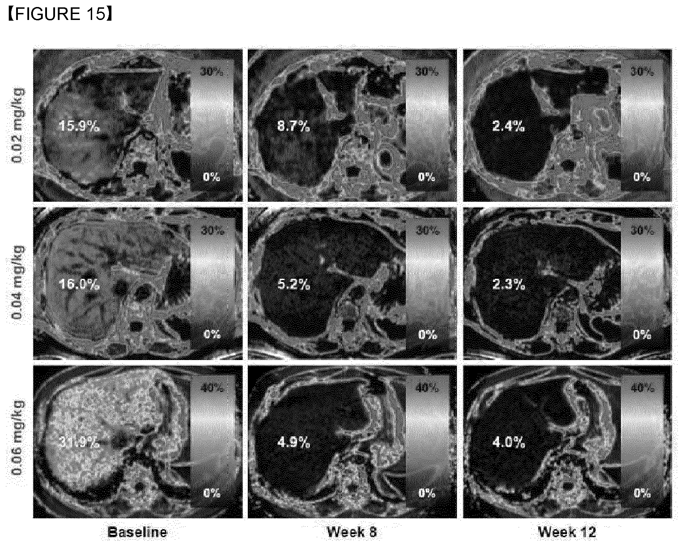

Liver fat content and hepatic steatosis levels of the patients with a non-alcoholic fatty liver disease were measured on Week 8 and Week 12 after initial administration by magnetic resonance imaging-derived proton density fat fraction (MRI-PDFF), and the results are shown in FIGS. 15 to 17.

Through the experiment, it was confirmed that the liver fat content may be reduced by administering the long-acting conjugate of the triple agonist according to the present invention to patients with a non-alcoholic fatty liver disease and thereby obtain therapeutic effects on the non-alcoholic fatty liver disease.

Based on the results, it was confirmed that stability, drug tolerance, and efficacy were obtained in the patients with a non-alcoholic fatty liver disease via parenteral administration (subcutaneous administration) once a week at a dose of 0.5 mg to 8 mg.

### Experimental Example 5: Experiment on Stability, Drug Tolerance, and Efficacy of Long-acting Conjugate of Triple Agonist in Patient with Non-alcoholic Fatty Liver Disease

According to the results of Experimental Example 4, the patients with a non-alcoholic fatty liver disease were classified into a group administered once a week at a dose of 2 mg, a group administered once a week at a dose of 4 mg, and a group administered once a week at a dose of 6 mg, followed by designing an experiment of Experimental Example 5, and the results were observed.

The long-acting conjugate of SEQ ID NO: 42, the effects of which were confirmed in Experimental Example 4, was administered to the patients with a non-alcoholic fatty liver disease once a week at a dose of 2 mg to 6 mg for 52 weeks.

Patients aged over 18 but less than 70, having a BMI of 18 kg/m² or more, diagnosed with liver fibrosis (fibrosis stages of F1-F3) and noncirrhotic non-alcoholic steatohepatitis (NASH), and an MRI-PDFF liver fat content of 8% or more were collected.

Specifically, the patients were classified into the following three administered groups and a placebo group and subcutaneously administered once a week for 52 weeks, and then stability (TEAE) was observed for 4 weeks after final administration:
- Group administered once a week at a dose of 2 mg
- Group administered once a week at a dose of 4 mg
- Group administered once a week at a dose of 6 mg

Liver fat content and hepatic steatosis levels of the patients with a non-alcoholic fatty liver disease were measured on Week 26 and Week 52 after initial administration by magnetic resonance imaging-derived proton density fat fraction (MRI-PDFF).

Fibro scan (echosens) was performed on Week 14, Week 26, Week 38, and Week 52 after initial administration, and blood lipid concentration (total cholesterol, LDL-C, HDL-C, VLDL-C, triglyceride, and free fatty acid), NASH biomarkers (Cytokeratin-18 M30/65 fragments, Enhanced Liver Fibrosis Score, Pro-C3, Non-invasive score 4, Fibrosis-4 index, and NAFLD Fibrosis Score), PK/PD analysis, and the following glucose metabolism parameters were identified on Week 14, Week 26, Week 38, and Week 52 after administration.

### <Glucose metabolism parameter>

- FPG (Fasting Plasma Glucose)
- fasting insulin
- Fasting C-Peptide
- HbA1c
- Insulin resistance: HOMA-IR (Homeostatic Model Assessment for Insulin Resistance)
- Insulin secretion: HOMA-B (Homeostatic Model Assessment for Insulin Secretion)

In order to evaluate the degree of alleviation of the non-alcoholic fatty liver disease, liver biopsy was conducted on Week 52 after initial administration, and the degree of alleviation was evaluated using NAFLD activity scores (0 to 8) and fibrosis scores (0 to 4) as shown in Table 4 below.

**Table 4**

| Item | Score | Content |
|---|---|---|
| Distribution of liver fat accumulation (steatosis grade, 0 to 3) | 0 | <5% |
| | 1 | 5-33% |
| | 2 | >33-66% |
| | 3 | >66% |
| Number of lobular inflammation (0 to 3) | 0 | No foci |
| | 1 | <2 foci per 200× field |
| | 2 | 2-4 foci per 200× field |
| | 3 | >4 foci per 200× field |
| Ballooning degeneration of hepatocytes (0 to 2) | 0 | None |
| | 1 | Few balloon cells |
| | 2 | Many cells/prominent ballooning |

The fibrosis score was obtained by evaluating Perisinusoidal Chicken-Wire Fibrosis, Portal Fibrosis, and Bridging Fibrosis using scores of 0 to 4.

In the present invention, when at least one of the following three items is satisfied, it may be determined that the non-alcoholic fatty liver disease is alleviated.
- Reduction in NAS by 2 or more
- Score of ballooning degeneration of hepatocytes of 0 or the score of number of lobular inflammation of 0 to 1
- Increase in fibrosis score by 1 or more

In accordance with the results of the experiments on stability, drug tolerance, and efficacy, it is suggested that patients with a non-alcoholic fatty liver disease receive parenteral administration once a week at a dose of 0.5 mg to 8 mg, preferably once a week at a dose of 2 mg to 6 mg, to obtain stability, drug tolerance, and efficacy.

The above description of the present invention is provided for the purpose of illustration, and it would be understood by those skilled in the art that various changes and modifications may be made without changing the technical conception and essential features of the present invention. Thus, it is clear that the above-described embodiments are illustrative in all aspects and do not limit the present invention. The various embodiments disclosed herein are not intended to be limiting, with the true scope and spirit being indicated by the following claims. The present invention is to be limited only by the terms of the appended claims, along with the full scope of equivalents to which such claims are entitled.

## Claims

1. A pharmaceutical composition for preventing or treating obesity or a non-alcoholic fatty liver disease, the composition comprising, as an active ingredient, a long-acting conjugate of a triple agonist represented by Formula 1 below and having activities to all of a glucagon receptor, a glucagon-like peptide-1 (GLP-1) receptor, and a glucose-dependent insulinotropic polypeptide (GIP) receptor,
wherein the long-acting conjugate of the triple agonist is parenterally administered to a patient with obesity or the non-alcoholic fatty liver disease once a week at a dose of 0.5 mg to 8 mg:
[Formula 1] X-L-F
wherein X is a peptide including an amino acid sequence of SEQ ID NOS: 1 to 102;
L is a linker including an ethylene glycol repeating unit;
F is an immunoglobulin Fc region; and
- is a covalent bond between X and L and between L and F.

2. The pharmaceutical composition of claim 1, wherein the long-acting conjugate of the triple agonist is parenterally administered to a patient with obesity or a non-alcoholic fatty liver disease once a week at a dose of 2 mg to 6 mg.

3. The pharmaceutical composition of claim 1, wherein the parenteral administration is subcutaneous administration.

4. The pharmaceutical composition of claim 1, wherein the patient with obesity has a body mass index (BMI) of 23 kg/m² or more.

5. The pharmaceutical composition of claim 1, wherein the patient with a non-alcoholic fatty liver disease has a liver fat content of 8% or more measured by magnetic resonance imaging-derived proton density fat fraction (MRI-PDFF).

6. The pharmaceutical composition of claim 1, wherein an individual administered with the pharmaceutical composition exhibits at least one of properties (a) to (f) below:
(a) weight loss;
(b) blood pressure decrease;
(c) visceral fat mass reduction in the liver;
(d) NAS decrease;
(e) reduction in ballooning degeneration of hepatocytes or the number of lobular inflammation; and
(f) fibrosis score decrease.

7. The pharmaceutical composition of claim 1, wherein the pharmaceutical composition is administered to an arm, thigh, or abdomen.

8. The pharmaceutical composition of claim 1, wherein the F is an IgG Fc region.

9. The pharmaceutical composition of claim 1, wherein the long-acting conjugate has a structure in which the Fc region is in a dimer form formed of two polypeptide chains and the peptide X is linked to only one of the two polypeptide chains of the Fc dimer in the long-acting conjugate.

10. The pharmaceutical composition of claim 9, wherein the polypeptide chain of the Fc dimer includes an amino acid sequence of SEQ ID NO: 123.

11. The pharmaceutical composition of claim 1, wherein the X includes an amino acid sequence selected from the group consisting of SEQ ID NOS: 21, 22, 27, 30 to 32, 34, 36, 37, 42, 43, 50 to 56, 58, 64 to 80, 83, 86, 91, 93, and 96 to 102.

12. The pharmaceutical composition of claim 1, wherein the X includes an amino acid sequence selected from the group consisting of SEQ ID NOS: 21, 22, 31, 32, 37, 42, 43, 50, 53, 64, 65, 66, 67, 68, 69, 70, 71, 72, 73, 75, 76, 77, 79, 96, 97, 98, 99, 100, 101, and 102.

13. The pharmaceutical composition of claim 1, wherein the X includes an amino acid sequence selected from the group consisting of SEQ ID NOS: 42, 43, and 50.

14. The pharmaceutical composition of claim 1, wherein the L is polyethylene glycol having a molecular weight of 1 kDa to 20 kDa.

15. The pharmaceutical composition of claim 1, wherein the non-alcoholic fatty liver disease is selected from the group consisting of non-alcoholic fatty liver (NAFL), non-alcoholic steatohepatitis (NASH), liver fibrosis, cirrhosis, and any combination thereof.
